# EUROPEAN PATENT SPECIFICATION

(11) **EP 3 849 706 B1**
(45) Date of publication and mention of the grant of the patent: **14.06.2023**
(21) Application number: 19769339.3
(22) Date of filing: 03.09.2019
(51) Int. Cl.: B01L 3/02, B01L 3/00, C12M 1/26

(54) **PIPETTE STRUCTURE AND METHODS UTILIZING SAME**
PIPETTENSTRUKTUR UND VERFAHREN ZUR VERWENDUNG DAVON
STRUCTURE DE PIPETTE ET PROCÉDÉS L'UTILISANT

(30) Priority: 11.09.2018 US 201862729626 P
(43) Date of publication of application: 21.07.2021
(73) Proprietor: Corning Incorporated, Corning, New York 14831 (US)
(72) Inventor: PARDO, Ana Maria del Pilar, Portsmouth, New Hampshire 03801 (US); MARTIN, Gregory Roger, Acton, Maine 04001 (US); TANNER, Allison Jean, Portsmouth, New Hampshire 03801 (US)
(74) Representative: Elkington and Fife LLP
(86) International application number: PCT/US2019/049283
(87) International publication number: WO 2020/055623

(56) References cited:
- WO-A1-01/68258
- WO-A1-2007/119448
- WO-A1-2016/069892
- WO-A1-2017/091540
- CN-U- 204 294 248
- CN-U- 206 334 690
- DE-A1-102010 040 681
- ES-U- 1 214 345
- US-A- 5 494 828
- US-A- 5 693 033
- US-A1- 2008 125 696
- US-A1- 2014 350 515
- US-A1- 2016 245 729
- US-B1- 6 497 155
- US-B2- 8 282 604

## Description

### CROSS-REFERENCE TO RELATED APPLICATION

### FIELD

The present disclosure relates generally to pipette structures, such as unitary measuring pipettes and extension tip devices for use with measuring pipettes, as well as methods utilizing such pipette structures.

### BACKGROUND

Pipettes are well-known tubular devices that usually have openings at both ends, and are designed to dispense measured quantities of liquids. Pipettes have had widespread usage in a number of industries where accurate measurement and delivery of fluids are required, particularly the medical and laboratory testing and analysis fields. Measuring pipettes typically embody straight glass or plastic tubes with one tapered end, and are calibrated into small divisions so that various amounts of liquid can be measured with the same pipette. Measuring pipettes include Mohr pipettes (with graduation marks that end before tapering begins proximate to the tip) and serological pipettes (with graduation marks that continue to a tapering region proximate to the tip).

Multiple different methods exist for fabricating pipettes, including (i) welding mouthpiece and tip components to a hollow tube, (ii) reheating a thick tube followed by drawing down and trimming the pipette at one or both ends to form a tip and mouthpiece, and (iii) molding with application of a pressure differential, including vacuum forming and blow molding.

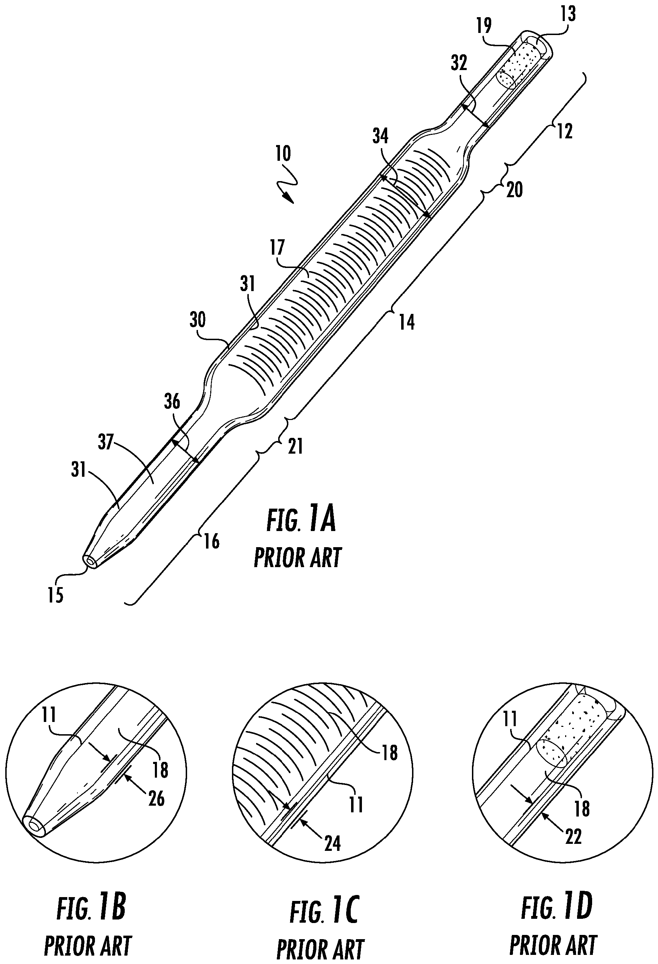

Examples of molding with application of a pressure differential according to method (iii) to form pipettes are disclosed in International Publication No. WO 2017/091540 A1 entitled "Unitary Serological Pipette and Methods of Producing the Same," which is assigned to Corning Incorporated. An exemplary pipette 10 that may be produced according to such a method is shown in FIG. 1A, with the pipette 10 including a mouth region 12, a body region 14, and a tip region 16, and with magnified portions of the foregoing regions shown in FIGS. 1B-1D, respectively. (FIGS. 1A-1D correspond to figures contained in International Publication No. WO 2017/091540 A1) Each of the mouth region 12, the body region 14, and the tip region 16 may have a corresponding wall thickness (namely, a mouth thickness 22, a body thickness 24, and a tip thickness 26) and a corresponding diameter (namely, a mouth diameter 32, a body diameter 34, and a tip diameter 36). FIGS. 1B-1D also show the pipette 10 as having an inner curved surface 11 that encloses a space 18. Referring to FIG. 1A, the pipette 10 includes a mouth 13 and a tip 15 that are aligned along a longitudinal axis, and includes a filter 19 proximate to the mouth 13. Optionally, the pipette 10 may have a mouth-body transition region 20 between the mouth region 12 and the body region 14, as well as a body-tip transition region 21 between the body region 14 and the tip region 16. In certain implementations, a substantially smooth inside surface 31 is provided in the transition regions 20, 21 to reduce retention of fluid and/or particulate material. The pipette 10 may also include a series of graduated volumetric markings 17 printed (or imprinted) along an outside surface 30 of (at least) the body region 14 to indicate a volume of liquid contained in the space 18 within the pipette 10. The pipette 10 may be sized to hold a particular volume of liquid (e.g., 1 mL, 2 mL, 5 mL, 10 mL, 25 mL, 50 mL, 100 mL, or another desired volume). The pipette 10 may be manufactured of any suitable materials, such as glass or polymers (e.g., polystyrene, polyethylene, or polypropylene).

Optionally, the mouth thickness 22, the tip thickness 26, or both the mouth thickness 22 and the tip thickness 26, may be similar to the body thickness 24. In certain implementations, one, some, or all of the mouth thickness 22, the tip thickness 26, and the body thickness 24 may be in a range of from 0.25 mm to 2.5 mm, or from 0.4 mm to 1.5 mm, or from 0.6 mm to 1.0 mm, or from 0.25 mm to about 0.5 mm, or from about 0.25 mm to about 0.5 mm. Enhanced thickness in the mouth and tip regions 12, 16 may provide certain advantages, such as by making such regions more resistant to damage or breakage during use. The mouth, body, and tip diameters 32, 34, 36 may each be measured externally (e.g., between opposing points on an outer surface of the pipette 10). Optionally, the body diameter 34 may be greater than either the mouth diameter 32 or the tip diameter 36. The specific body diameter 34 may depend on the volume of liquid the pipette 10 is sized to hold. In certain instances, the body diameter may be in a range of from about 4.0 mm to about 25.0 mm.

Fabrication of the pipette 10 by molding with application of a pressure differential may include supplying a heated parison (e.g., a tube or preform, typically in the shape of a hollow cylinder) into a mold, and creating a differential pressure between an interior and an exterior of the parison to cause the parison to expand and conform to a cavity of the mold. Optionally the heated parison may be extruded directly into the mold. A parison may be manufactured of any suitable material (including polymers such as polystyrene and polypropylene, or glass), for example, by extruding a polymer melt to form a hollow cylindrical tube. Differential pressure across a wall of a parison may be created by either supplying pressurized gas (e.g., compressed air at 0.05 to 1.5 MPa) into an interior of the parison, or by generating subatmospheric pressure conditions (also known as vacuum conditions, e.g., at a pressure of 0.01 to 0.09 MPa) along surfaces defining the cavity of the mold. When the expanded material (now embodied in a pipette) has cooled sufficiently, the mold is opened, the pipette is ejected, and the mold may receive another heated parison (e.g., by extrusion into the mold) to repeat the process. Thereafter, a filter 19 (typically comprising a fibrous material) may be inserted into the mouth 13 of the pipette 10 to be retained within the mouth region 12.

Pipettes may be useful for transferring materials (e.g., liquids) in various commercial and laboratory contexts, including cell culture apparatuses. Traditional two-dimensional (2D) cell culture involves layer-type growth of cells on a substrate (e.g., agar in a petri dish). Three dimensional (3D) cell culture involves the growth of cells in an artificially-created environment that allows aggregates or clusters of cells (e.g., spheroids) to grow and/or interact primarily with each other in all three dimensions - as compared with 2D cell culture in which cells interact primarily with the substrate. 3D cell culture represents an improvement over 2D cell growth methods for at least the reason that the 3D conditions more accurately model the *in vivo* environment in terms of cellular communication and development of extracellular matrices, since cells interact with one another rather than attaching to the substrate.

One issue with spheroid based assays is that the assay results typically vary with the size of the spheroid. For example, variations in variables (e.g., seeding density and growth time) from system to system may affect assay repeatability from system to system or from well to well within a given system. As the density of cells grown in cell culture apparatus increases, larger volumes of cell culture media or more frequent exchange of cell culture media may be needed to maintain the cells. However, increased frequency of media exchange can be inconvenient. In addition, increased volumes of cell culture media can lead to undesirably increased height of media above the cultured cells. As the media height increases, gas exchange rate for the cells through the media decreases.

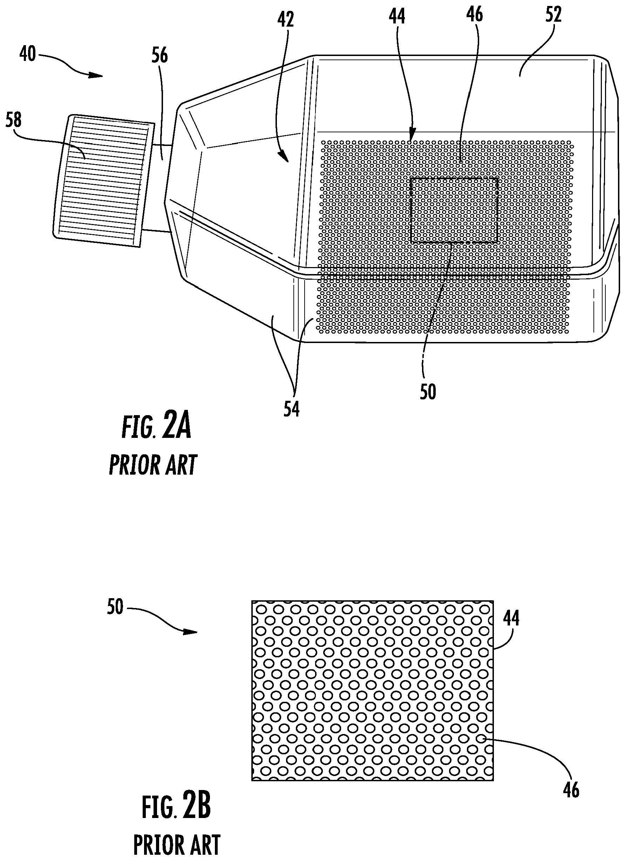

Exemplary cell culture flasks or housings including arrays of microwells suitable for culture of 3D cell aggregates are disclosed in International Publication No. WO 2016/069892 A1 entitled "Devices and Methods for Generation and Culture of 3D Cell Aggregates of Corning Incorporated. A first exemplary cell culture housing from the foregoing publication is reproduced in FIG. 2A of the present application. The cell culture housing 40 encloses a cell culture chamber 42, which bounds a structured surface 44 defining an array of microwells 46 (also shown in FIG. 2B, which is a magnified view of a central portion 50 of the structured surface of FIG. 2A) along the bottom of the cell culture chamber 42. The cell culture housing 40 further includes a top wall 52, and side walls 54 extending upward from the structured surface 44 to the top wall 52. The cell culture housing 40 additionally includes a port 56, which may be embodied in an opening having a screw cap 58. In use, at least a portion of the cell culture chamber 42 may be filled with a cell culture medium at a desired height of cell culture medium above cells contained in the microwells 46.

Housings for culturing 3D cell aggregates have different handling requirements than vessels suitable for 2D cell culturing. In the context of high-density 3D cell culture housings, it is necessary to periodically remove spent cell culture medium without removing cell aggregates/spheroids. Separately, it is frequently necessary to collect samples of selected cell aggregates/spheroids from 3D cell culture housings without disrupting the entire culture.

Conventional serological pipettes (e.g., 2mL capacity) are commonly used for aspirating media from tissue culture flasks. Use of such pipettes is a well-established method for adherent 2D cell cultures, but cell culture housings including arrays of microwells (such as shown in FIG. 2A) have a positional limitation. If a structured surface defining microwells is tipped at too great an angle from horizontal, then the cell aggregates/spheroids will be displaced from the microwells, thereby ruining the culture. Standard serological pipets can easily disturb and/or remove cell aggregates. Pipette tip aspirators that are commonly used on petri dishes and well plates cannot be used with cell culture housings due to sterility concerns. Large bore pipettes that are useable to collection of aggregates/ spheroids from well plates or petri dishes may be ill-suited for transferring materials to and from cell culture housings, due at least in part to the geometry of a cell culture housing port relative to microwells defined in a structured surface.

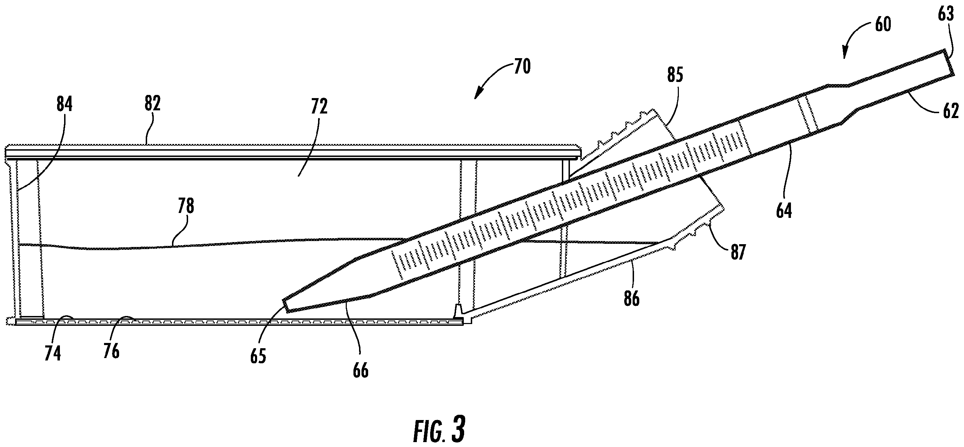

FIG. 3 is a side, partial cross-sectional view of another cell culture housing 70 receiving a portion of a conventional serological pipette 60 therein. The cell culture housing 70 encloses a cell culture chamber 72 containing a cell culture medium 78, with the bottom of the cell culture chamber 72 containing a structured surface 74 defining an array of microwells 76. The cell culture housing 70 further includes a top wall 82, side walls 84 extending upward from the structured surface 74 to the top wall 82, and a tubular neck 86 that extends outward and upward to define a port opening 85. A center axis of the tubular neck 86 may be angled upward at an acute angle (e.g., in a range of from 20 degrees to 45 degrees). The tubular neck 86 includes external threads 87 suitable for receiving a threaded cap (not shown). The serological pipette 60 includes a mouth region 62, a tubular body region 64, and a tip region 66, with the foregoing regions 62, 64, 66 being arranged in sequence between a mouth 63 and a tip 65 that are aligned along a longitudinal axis. Graduated markings are provided along at least a portion of the tubular body region 64 to aid in measuring contents of the pipette 60. As shown in FIG. 3, the pipette 60 is inserted through the tubular neck 86 at a shallow angle, as dictated by the shallow angle between the tubular neck 86 and a bottom of the cell culture chamber 72. Due to this shallow angle positioning of the pipette 60, the tip 65 is arranged decidedly non-parallel relative to the structured surface 74, thereby rendering it difficult to extract samples of cell aggregates from desired microwells 76 - particularly from microwells arranged a long distance from the port opening 85. The small diameter of an opening defined in the tip 65 of the pipette 60 also limits the ability to simultaneously extract cell aggregates from multiple adjacent microwells 76. Although larger pipettes may be provided with larger tip openings, the use of larger diameter pipettes with a cell culture housing 70 as illustrated in FIG. 3 may be precluded by the need to accommodate maneuverability of a pipette body relative to the port opening 85.

Given the foregoing, there is a need for apparatuses free of the aforementioned shortcomings, as well as a need for improved material transfer methods for use with cell culture housings including arrays of microwells.

CN206334690 U discloses a pipette tip with a main body part separated from a tapered end part by a flexible bellows-like portion.

US6497155 B1 discloses a particle-retrieval device with a receiver tube, vacuum-flow providing means and particle-disengaging means. In some configurations, the device comprises a bellows whose collapse is used to detect when a particle is engaged to a tip of the device. In some configurations, a tip of the device includes a flared end.

WO01/68258 A1 discloses pipette or pipette tip having in which the open distal end of the pipette or pipette tip is conformed such that a plane passing across the distal opening is not perpendicular to the longitudinal axis of the pipette's body.

### SUMMARY

The invention is defined in the appended claims. In a first aspect, a pipette structure is as defined in claim 1.

In certain embodiments, the pipette structure is embodied in a unitary measuring pipette, wherein: the first body section comprises a tubular body of the measuring pipette; the second body section comprises a tip region of the measuring pipette; and the tubular body is arranged between the tip region and a mouth region of the measuring pipette.

In certain embodiments, the pipette structure is embodied in an extension tip device for use with a measuring pipette, wherein the first body section defines a cavity configured to receive a tip region of the measuring pipette. In certain embodiments, the cavity is bounded by an inner surface, and the inner surface defines an annular recess configured to receive a sealing ring to promote sealing and/or retention between the extension tip device and the tip region of the measuring pipette.

In certain embodiments, at least a portion of the second body section proximate to the sample introduction end comprises a central longitudinal axis, and a tip of the sample introduction end is offcut at an angle non-perpendicular to the central longitudinal axis. In certain embodiments, the tip of the sample introduction end is offcut at an angle in a range of from 5 degrees to 45 degrees (or in a range of from 10 degrees to 40 degrees, or in a range of from 15 to 35 degrees) from perpendicular to the central longitudinal axis.

In certain embodiments, a tip of the sample introduction end comprises a primary width dimension and a transverse width dimension, wherein the primary width dimension is at least two times greater than the transverse width dimension. In certain embodiments, the primary width dimension at the sample introduction end is larger than the width dimension at the junction, and the transverse width dimension at the sample introduction end is smaller than the width dimension at the junction.

In certain embodiments, the first body section and the second body section comprise at least one polymeric material. In certain embodiments, a welded interface is provided between the first body section and the second body section along at least a portion of the junction.

In certain embodiments, the first body section and the second body section are configured or are configurable to permit a central longitudinal axis of the second body section to be oriented at an angle in a range of from 100 degrees to 170 degrees (or in a subrange of 110 to 160 degrees, or in a subrange of from 115 to 155 degrees, or in a subrange of from 115 to 145 degrees) relative to a central longitudinal axis of the first body section.

In a second aspect, there is provided a method for transferring at least one material between (i) an interior of a cell culture housing that includes a structured surface defining an array of microwells suitable for three-dimensional culture of multi-cell aggregates and (ii) an environment external to the cell culture housing as defined in claim 7.

In certain embodiments, the at least one material comprises cell culture medium within the interior of the cell culture housing, and the method further comprises maintaining the sample introduction end in a non-contacting relationship relative to the structured surface in a manner to preferentially remove cell culture medium from the interior of the cell culture housing while reducing a likelihood of removing cell multi-cell aggregates from the array of microwells during said extracting of the at least one material from the interior of the cell culture housing.

In certain embodiments, the at least one material comprises the multi-cell aggregates, and the method further comprises maintaining the sample introduction end proximate to one or more selected microwells of the array of microwells in a manner to preferentially remove at least some multi-cell aggregates from the one or more selected microwells during said extracting of the at least one material from the interior of the cell culture housing.

Additional features and advantages of the subject matter of the present disclosure will be set forth in the detailed description which follows, and in part will be readily apparent to those skilled in the art from that description or recognized by practicing the subject matter of the present disclosure as described herein, including the detailed description which follows, the claims, as well as the appended drawings.

It is to be understood that both the foregoing general description and the following detailed description present embodiments of the subject matter of the present disclosure, and are intended to provide an overview or framework for understanding the nature and character of the subject matter of the present disclosure as it is claimed. The accompanying drawings are included to provide a further understanding of the subject matter of the present disclosure, and are incorporated into and constitute a part of this specification. The drawings illustrate various embodiments of the subject matter of the present disclosure and together with the description serve to explain the principles and operations of the subject matter of the present disclosure.

### BRIEF DESCRIPTION OF THE DRAWINGS

The following is a description of the figures in the accompanying drawings. The figures are not necessarily to scale, and certain features and certain views may be show exaggerated in scale or in schematic, in the interest of clarity or conciseness.
FIG. 1A is a perspective view illustration of a unitary measuring pipette with graduation marks.
FIGS. 1B-1D provide magnified perspective views of a mouth region, a body region, and a tip region, respectively, of the pipette of FIG. 1A.
FIG. 2A is a perspective view of an exemplary cell culture housing enclosing a cell culture chamber that bounds a lower structured surface defining an array of microwells suitable for 3D cell culture.
FIG. 2B is a magnified portion of the lower structured surface of FIG. 2A.
FIG. 3 is a side, partial cross-sectional view of a cell culture housing receiving a portion of a conventional serological pipette therein.
FIG. 4A is a front elevational view of a serological pipette, including a second body section having an increased primary width (and a reduced transverse width) relative to a first tubular body section thereof, with a non-linear elbow transition (shown in FIG. 4C) between the first and second body sections.
FIGS. 4B and 4C provide rear elevational and right side elevational views, respectively, of the pipette of FIG. 4A, with dashed lines illustrating boundaries of fluid passages internal to the pipette.
FIGS. 4D and 4E provide rear side and right side elevational views, respectively, of an alternative serological pipette similar to the pipette of FIGS. 4A-4C, with a tip of the sample introduction end being offcut at an angle non-perpendicular to a central longitudinal axis of the second body section, and with FIG. 4E including dashed lines illustrating boundaries of fluid passages internal to the pipette.
FIG. 5A is a front elevational view of a serological pipette, including a second body section having an increased primary width (and a reduced transverse width) relative to a first tubular body section thereof, with the first and second body sections being collinearly arranged.
FIGS. 5B and 5C provide front elevational and right side elevational views, respectively, of the pipette of FIG. 5A, with dashed lines illustrating boundaries of fluid passages internal to the pipette.
FIG. 5D is a bottom plan view of the pipette of FIGS. 5A-5C.
FIG. 6A is a front elevational view of a serological pipette including a second body section having an increased primary width (and an increased transverse width) relative to a first tubular body section thereof, with the first and second body sections being collinearly arranged.
FIGS. 6B and 6C provide front elevational and right side elevational views, respectively, of the pipette of FIG. 6A, with dashed lines illustrating boundaries of fluid passages internal to the pipette.
FIG. 6D is a bottom plan view of the pipette of FIGS. 6A-6C, illustrating a sample introduction end of the pipette as being round in shape.
FIG. 6E is a bottom plan view of an alternative pipette similar to the embodiment of FIGS. 6A-6D, illustrating a sample introduction end of the pipette as being substantially square in shape.
FIGS. 7A and 7B provide front elevational and right side elevational views, respectively, of a serological pipette including a second body section having an increased primary width (and an increased transverse width) relative to a first tubular body section thereof, with a bellows joint in a straight configuration arranged between the first and second body sections.
FIG. 7C is a right side elevational view of the pipette of FIGS. 7A-7B, following pivotal movement of the bellows joint to cause the first and second body sections to be non-collinearly arranged.
FIG. 7D is a right side elevational view of an alternative serological pipette similar to the pipette of FIGS. 7A-7C, with a tip of the sample introduction end being offcut at an angle non-perpendicular to a central longitudinal axis of the second body section, and with a bellows joint in a straight configuration arranged between the first and second body sections.
FIG. 7E is a right side elevational view of the pipette of FIG. 7D, following pivotal movement of the bellows joint to cause the first and second body sections to be non-collinearly arranged.
FIGS. 8A and 8B provide front elevational and right side elevational views, respectively, of a serological pipette, including a second body section having a constant primary width (and a reduced transverse width) relative to a first tubular body section thereof, with a bellows joint in a straight configuration arranged between the first and second body sections.
FIG. 8C is a bottom plan view of the pipette of FIGS. 8A-8B.
FIG. 8D is a right side elevational view of an alternative serological pipette similar to the pipette of FIGS. 8A-8C, with a tip of the sample introduction end being offcut at an angle non-perpendicular to a central longitudinal axis of the second body section.
FIGS. 9A and 9B provide front and right side cross-sectional views, respectively, of an extension tip device including a first body section configured to receive a tip region of a measuring pipette, and including a second body section having an increased primary width (and a reduced transverse width) relative to the first body section.
FIG. 9C is a bottom plan view of the extension tip device of FIGS. 9A-9B.
FIG. 9D is a front, partial cross-sectional view of the extension tip device of FIGS. 9A-9C, with a tip region of a measuring pipette received in a cavity of the first body section of the extension tip device.
FIGS. 10A and 10B provide front and right side cross-sectional views, respectively, of an extension tip device substantially similar to the extension tip device of FIGS. 9A-9C, modified to include an annular recess that receives a sealing ring (e.g., an O-ring) to promote sealing and/or retention between the extension tip device and the tip region of the measuring pipette.
FIG. 10C is a front, partial cross-sectional view of the extension tip device of FIGS. 10A-10B, with a tip region of a measuring pipette received in a cavity of the first body section of the extension tip device.
FIGS. 11A and 11B provide front and right side cross-sectional views, respectively, of an extension tip device including a first body section configured to receive a tip region of a measuring pipette, and including a second body section having an increased primary width (and a reduced transverse width) relative to the first body section, with a bellows joint in a straight configuration arranged between the first and second body sections.
FIG. 11C is a bottom plan view of the extension tip device of FIGS. 11A-11B.
FIG. 11D is a front, partial cross-sectional view of the extension tip device of FIGS. 11A-11B, with a tip region of a measuring pipette received in a cavity of the first body section of the extension tip device.
FIGS. 12A and 12B provide front and right side cross-sectional views, respectively, of an extension tip device substantially similar to the extension tip device of FIGS. 11A-11C, modified to include an annular recess that receives a sealing ring (e.g., an O-ring) to promote sealing and/or retention between the extension tip device and the tip region of the measuring pipette.
FIG. 12C is a front, partial cross-sectional view of the extension tip device of FIGS. 12A-12B, with a tip region of a measuring pipette received in a cavity of the first body section of the extension tip device.
FIG. 13 is a right side elevational view of an extension tip device similar to the extension tip device of FIGS. 9A-9C, with a tip of the sample introduction end being offcut at an angle non-perpendicular to a central longitudinal axis of the second body section.
FIG. 14 is a right side elevational view of an extension tip device similar to the extension tip device of FIGS. 9A-9C, with a non-linear elbow transition arranged between the first and second body sections.
FIG. 15 is a right side elevational view of an extension tip device according to one embodiment similar to the extension tip device of FIGS. 11A-11C, with a non-linear elbow transition arranged between the first and second body sections proximate to the bellows joint, and with a tip of the sample introduction end being offcut at an angle non-perpendicular to a central longitudinal axis of the second body section.
FIG. 16A is a front elevational view of an extension tip device similar to the extension tip device of FIGS. 11A-11C, with the second body section having an increased primary width and an increased transverse width relative to a width of the first body section.
FIG. 16B is a bottom plan view of the extension tip device of FIG. 16A, illustrating a sample introduction end of the pipette as being round in shape.
FIG. 16C is a bottom plan view of an alternative extension tip device similar to the embodiment of FIG. 16B, illustrating a sample introduction end of the extension tip device as being substantially square in shape.
FIGS. 17A and 17B provide front and right side elevational views, respectively, of an extension tip device including a second body section having a constant primary width (and a reduced transverse width) relative to a first body section thereof, with a bellows joint in a straight configuration arranged between the first and second body sections.
FIG. 17C is a bottom plan view of the extension tip device of FIGS. 17A-17B.
FIG. 17D is a right side elevational view of an alternative extension tip device similar to the embodiment of FIGS. 17A-17C, with a tip of the sample introduction end being offcut at an angle non-perpendicular to a central longitudinal axis of the second body section.
FIG. 18A is a side, partial cross-sectional view of a cell culture housing receiving therein a portion of serological pipette , with the pipette including a bellows joint permitting pivotal movement between first and section body sections of the pipette, with the first and second body sections being non-collinearly arranged, and with a sample introduction end of the pipette being offcut and positioned proximate to microwells in a manner to preferentially remove multi-cell aggregates from one or more selected microwells.
FIG. 18B is a side, partial cross-sectional view of the cell culture housing and pipette of FIG. 18A, with the first and second body sections being collinearly arranged, and with the sample introduction end of the pipette being spaced apart from the structured surface of the cell culture housing and positioned to preferentially remove cell culture medium from the interior of the cell culture housing while reducing a likelihood of removing cell multi-cell aggregates from the array of microwells.
FIG. 19A is a side, partial cross-sectional view of a cell culture housing receiving therein an extension tip device into which a tip region of a serological pipette is received, with the extension tip device including a bellows joint permitting pivotal movement between first and section body sections thereof with the first and second body sections being non-collinearly arranged, and with a sample introduction end of the extension tip device being offcut and positioned proximate to microwells in a manner to preferentially remove multi-cell aggregates from one or more selected microwells.
FIG. 19B is a side, partial cross-sectional view of the cell culture housing, extension tip device, and serological pipette of FIG. 19A, with the first and second body sections being collinearly arranged, and with the sample introduction end of the extension tip device being spaced apart from the structured surface of the cell culture housing and positioned to preferentially remove cell culture medium from the interior of the cell culture housing while reducing a likelihood of removing cell multi-cell aggregates from the array of microwells.

### DETAILED DESCRIPTION

The present disclosure relates to a pipette structure including first and second body sections connected at a junction and including first and second fluid passages, with the pipette structure including (i) a bellows joint permitting pivotal movement between the first and second body sections, and/or (ii) the second fluid passage of the second body section includes greater width dimension at a sample introduction end than a width dimension at the junction. Such a pipette structure may be embodied in a unitary measuring pipette or an extension tip device for a measuring pipette.

The foregoing features (i) and (ii) may aid in transferring material between an interior of a cell culture housing (e.g., including a structured surface defining an array of microwells suitable for three-dimensional culture of multi-cell aggregates) and an environment external to the cell culture housing. Presence of a bellows j oint permitting pivotal movement between first and second body sections permits the angle between body sections to be varied as desired. Such angular variation may be useful, for example, to target different microwell regions along a structured surface of a cell culture housing (such as the cell culture housings illustrated in FIGS. 2A and 3). Alternatively, such angular variation may be useful to either position a sample introduction end away from microwells to preferentially remove cell culture medium from the interior of a cell culture housing during a material extraction step, or to position a sample introduction end proximate to one or more selected microwells to preferentially remove at least some multi-cell aggregates during a material extraction step.

Presence of a second fluid passage having a non-uniform width between the sample introduction end and the junction, with a width dimension at the sample introduction end that is greater than a width dimension at the junction, may beneficially permit multi-cell aggregates from multiple adjacent microwells of a cell culture housing to be extracted simultaneously, without requiring large diameter tubular pipette body section that would inhibit maneuverability of a pipette relative to the port opening of a cell culture housing (such as the cell culture housings illustrated in FIGS. 2A and 3).

In combination with both of the foregoing features (i) and (ii), pipette structures as according to the present disclosure herein may include additional features. Certain embodiments may include features to enhance an ability of a sample introduction end of a pipette structure to access desired regions of a cell culture housing having geometric limitations according to the cell culture housings of FIGS. 2A and 3. For example, in certain embodiments a tip of a sample introduction end is offcut at an angle (e.g., from 5 degrees to 45 degrees or any other angular range specified herein) non-perpendicular to a central longitudinal axis of a second body section proximate to the sample introduction end. In certain embodiments, a pipette structure may include a permanent bend between first and second body sections.

In certain embodiments, pipette structures as disclosed herein may be produced from glass or polymeric materials by one or more methods such as molding (including vacuum forming and/or blow molding), welding of prefabricated (e.g., extruded or molded) components, and/or other fabrication techniques. Welding of polymeric materials may include ultrasonic welding, laser welding, and/or solvent welding (also referred to as solvent cementing). In certain embodiments, all components of a pipette structure may be compositionally the same.

Following fabrication, pipette structures as disclosed herein may be sterilized and packaged in suitable sterile packaging in preparation for delivery to a user. When an extension tip device is provided, such a device may beneficially be used with conventional pipettes without compromising sterility. In certain embodiments, multiple identical extension tip devices as disclosed herein may be configured to at least partially nest within one another when stacked together. Such an arrangement may provide packaging efficiency when multiple extension tip devices are stacked in a package (e.g., a sterilized package) or dispenser.

Various features and aspects of pipette structures as disclosed herein will be apparent upon review of the accompanying figures and the descriptions thereof.

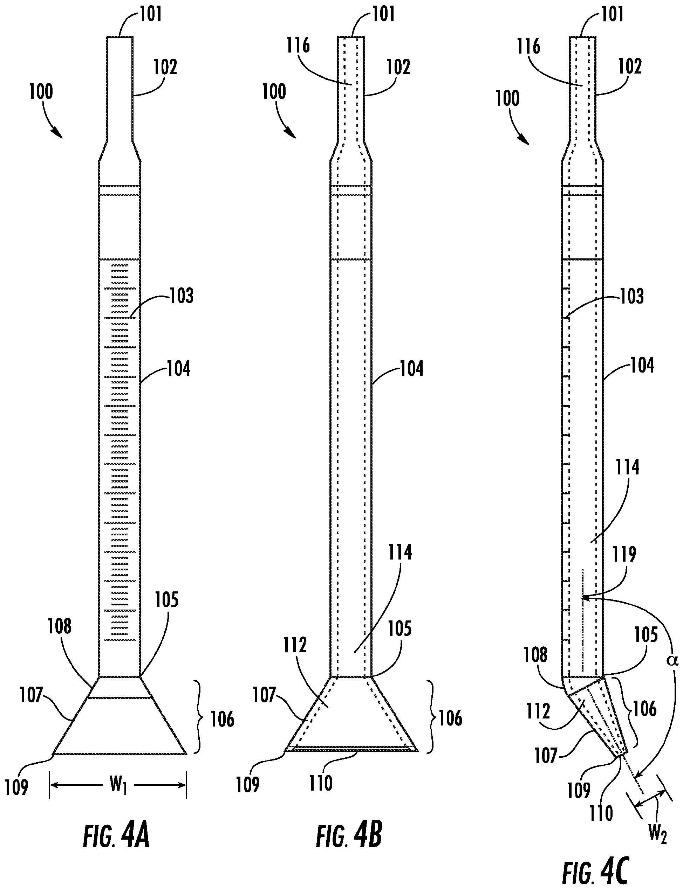

FIGS. 4A-4C illustrate a serological pipette 100 including a mouthpiece 102 proximate to a mouth end 101, a first body section 104 that is generally tubular in shape, and a second body section 106 that is proximate to a sample introduction end 109. The first body section 104 includes a series of graduated markings 103, and has a generally constant diameter. A non-linear elbow transition 108 (visible in FIGS. 4A and 4C) is arranged at a junction 105 (optionally including a welded interface) between the first and second body sections 104, 106. The second body section 106 includes a wall structure 107 bounding a fluid passage 112 (i.e., a second fluid passage 112) that opens to a sample introduction port 110 at the sample introduction end 109. Corresponding fluid passages 114, 116 are defined in the first body section 104 and the mouthpiece 102 (as well as in the elbow transition 108), and are in fluid communication with the fluid passage 112 defined in the second body section 106. The fluid passages 114, 112 defined in the first and second body sections 104, 106, respectively, may be referred to hereinafter as the first fluid passage 114 and the second fluid passage 112.

Various sections of the pipette 100 include a primary width dimension Wi (shown in FIG. 4A) and a transverse width dimension W₂ (shown in FIG. 4C) that may be orthogonal to the primary width dimension Wi. As shown in FIGS. 4A and 4B, the second body section 106 has a non-uniform primary width that increases with proximity to the sample introduction end 109 and that is greater than that of the first body section 104, and the second fluid passage 112 has a primary width that increases with proximity to the sample introduction end 109 and that is greater than that of the first fluid passage 114. Referring to FIG. 4C, the second body section 106 has a non-uniform transverse width that decreases with proximity to the sample introduction end 109 and that is smaller than that of the first body section 104, and the second fluid passage 112 has a transverse width that decreases with proximity to the sample introduction end 109 and that is smaller than that of the first fluid passage 114. Presence of the second fluid passage 112 having a non-uniform width between the sample introduction end 109 and the junction 105, with a primary width dimension at the sample introduction end 109 that is greater than a primary width dimension at the junction 105, may beneficially permit the pipette 100 to extract multi-cell aggregates from multiple adjacent microwells of a cell culture housing simultaneously.

As shown in FIG. 4C, the first body section 104 has a central longitudinal axis 119 that is non-parallel to a central longitudinal axis 118 of the second body section 106, with such axes 119, 118 being oriented an angle α relative to one another. The angle α may be in a range of from 100 degrees to 170 degrees (or in a range of from 110 degrees to 160 degrees, or in a range of from 115 degrees to 155 degrees, or in a range of from 120 degrees to 150 degrees). This angle α may have a complementary angle, serving as a transition angle for the non-linear elbow transition 108, wherein the transition angle may be in a range of from 10 degrees to 80 degrees (or any other range complementary to the ranges for the angle α outlined above).

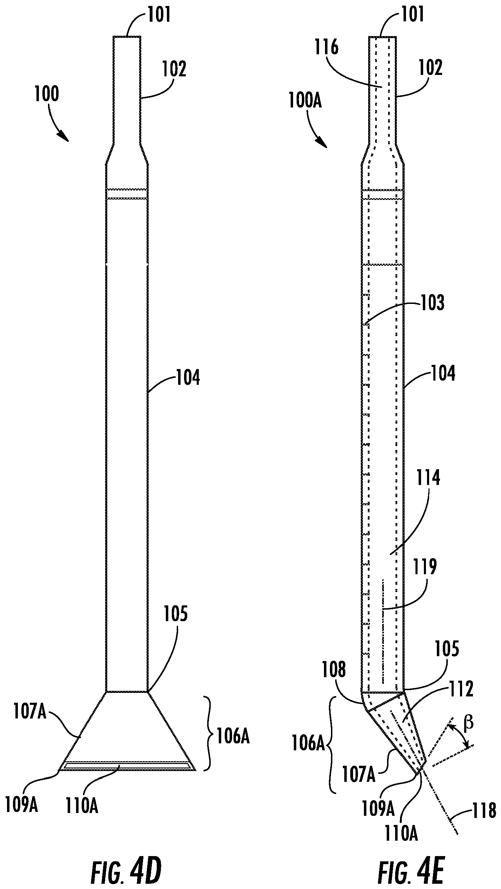

In certain embodiments, a pipette as disclosed herein may include a tip of a sample introduction end that is offcut at an angle non-perpendicular to a central longitudinal axis of a second body portion. Such an arrangement is shown in FIGS. 4D and 4E. FIGS. 4D and 4E provide rear side and right side elevational views, respectively, of an alternative serological pipette 100A similar to the pipette 100 of FIGS. 4A-4C. The pipette 100A includes a mouthpiece 102 proximate to a mouth end 101, a first body section 104 having a series of graduated markings 103, and a second body section 106A that is proximate to a sample introduction end 109A. A non-linear elbow transition 108 is arranged at a junction 105 between the first and second body sections 104, 106A. The second body section 106A includes a wall structure 107A bounding a fluid passage 112 (i.e., a second fluid passage) that opens to a sample introduction port 110A at the sample introduction end 109. Corresponding fluid passages 114, 116 are defined in the first body section 104 and the mouthpiece 102. The second body section 106A has a non-uniform primary width that increases with proximity to the sample introduction end 109 and that is greater than that of the first body section 104, and the second fluid passage 112 has a primary width that increases with proximity to the sample introduction end 109 and that is greater than that of the first fluid passage 114. The second body section 106A has a non-uniform transverse width that decreases with proximity to the sample introduction end 109A and that is smaller than that of the first body section 104, and the second fluid passage 112 has a transverse width that decreases with proximity to the sample introduction end 109A and that is smaller than that of the first fluid passage 114. The first body section 104 has a central longitudinal axis 119 that is non-parallel to a central longitudinal axis 118 of the second body section 106A. As shown in FIG. 4E, a tip of the sample introduction end 109A is offcut at an angle β that is non-perpendicular to the central longitudinal axis. The tip of the sample introduction end 109A can be offcut at an angle in a range of from 5 degrees to 45 degrees (or in a range of from 10 degrees to 40 degrees, or in a range of from 15 to 35 degrees) from perpendicular to the central longitudinal axis 118 of the second body section 106A. This offcut of the sample introduction end 109A may facilitate contacting the sample introduction end 109A and may promote more conformal contact between the pipette 100A and a structured surface of a cell culture housing as disclosed previously herein, to extract multi-cell aggregates from microwells of such a housing.

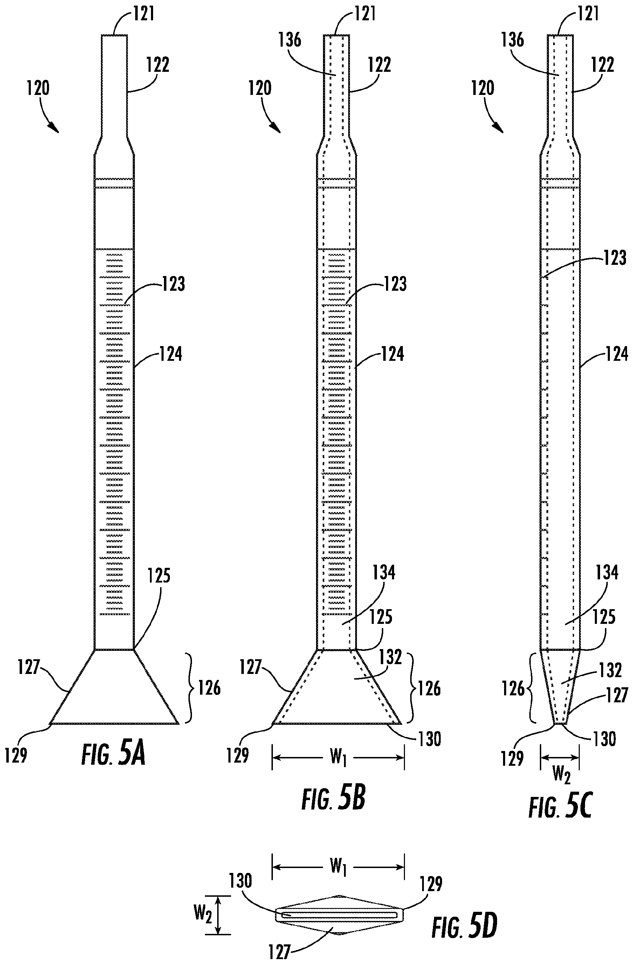

In certain embodiments, a pipette as disclosed herein may include a second body section that is collinearly arranged with a first body section, while including other features as disclosed herein. FIGS. 5A-5D illustrate a serological pipette 120 similar to the pipette 100 of FIGS. 4A-4C, but lacking a non-linear elbow transition between first and second body sections. The pipette 120 includes a mouthpiece 122 proximate to a mouth end 121, a first body section 124 having a series of graduated markings 123, and a second body section 126 that is proximate to a sample introduction end 129. A direct junction 125 is provided between the first and second body sections 124, 126. The second body section 126 includes a wall structure 127 bounding a fluid passage 132 (i.e., a second fluid passage) that opens to a sample introduction port 130 at the sample introduction end 129. Corresponding fluid passages 134, 136 are defined in the first body section 124 and the mouthpiece 122. The second body section 126 has a non-uniform primary width (along primary width dimension W₁) that increases with proximity to the sample introduction end 129 and that is greater than that of the first body section 124, and the second fluid passage 132 has a primary width that increases with proximity to the sample introduction end 129 and that is greater than that of the first fluid passage 134. The second body section 126 has a non-uniform transverse width (along transverse width dimension W₂) that decreases with proximity to the sample introduction end 129 and that is smaller than that of the first body section 124, and the second fluid passage 132 has a transverse width that decreases with proximity to the sample introduction end 129 and that is smaller than that of the first fluid passage 134. Longitudinal axes of the first and second body sections 124, 126 are collinearly arranged.

In various embodiments described previously herein, pipettes included second body portions with increasing primary width and decreasing secondary width with proximity to a sample introduction end. In certain embodiments, a pipette may include a second body portion having a primary width and a secondary width that both increase with proximity to a sample introduction end.

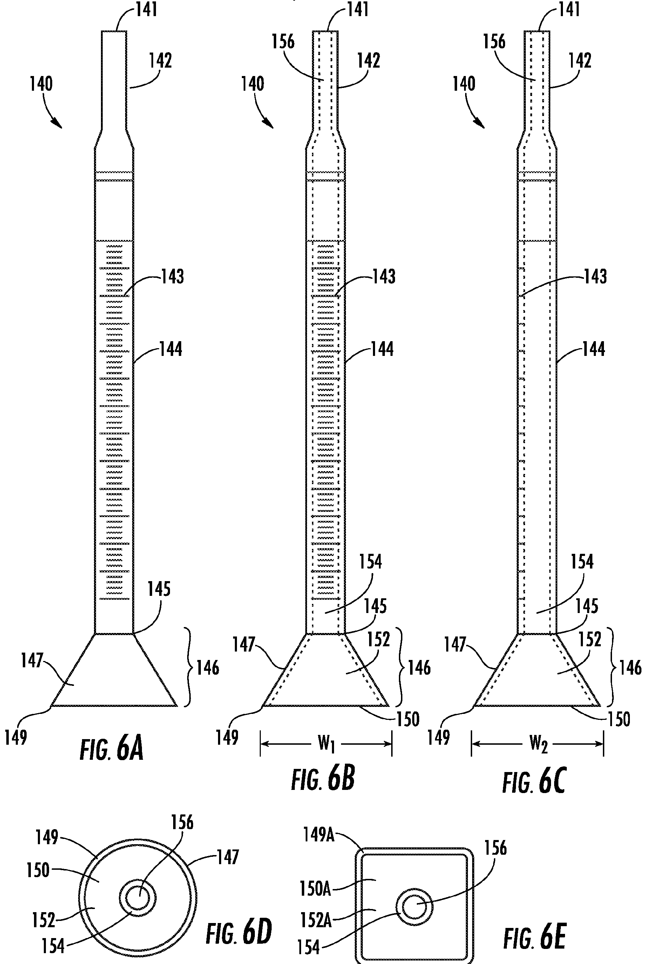

FIGS. 6A-6D illustrate a serological pipette 140 similar to the pipette 120 of FIGS. 5A-5D, with a second body portion having a primary width and a secondary width that both increase with proximity to a sample introduction end. The pipette 140 includes a mouthpiece 142 proximate to a mouth end 141, a first body section 144 having a series of graduated markings 143, and a second body section 146 that is proximate to a sample introduction end 149. A direct junction 145 is provided between the first and second body sections 144, 146. The second body section 146 includes a wall structure 147 bounding a fluid passage 152 that opens to a sample introduction port 150 at the sample introduction end 149. Corresponding fluid passages 154, 156 are defined in the first body section 144 and the mouthpiece 142. The second body section 146 has a non-uniform primary width along both the primary width dimension Wi and the transverse width dimension W₂ that increases with proximity to the sample introduction end 149, and that is greater than that of the first body section 144. Longitudinal axes of the first and second body sections 144, 146 are collinearly arranged. As shown in FIG. 6D, the wall structure 147 and a fluid passage 152 may be frustoconical in shape with a round cross-section. It is noted that a wall structure and fluid passage may have any suitable cross-sectional shape. For example, FIG. 6E is a bottom plan view of an alternative pipette similar to the pipette of FIGS. 6A-6D, illustrating a sample introduction end 149A, a sample introduction port 150A, and a fluid passage 152A all as being substantially square in shape, in combination with first and second fluid passages 154, 156 both having round cross-sectional shapes.

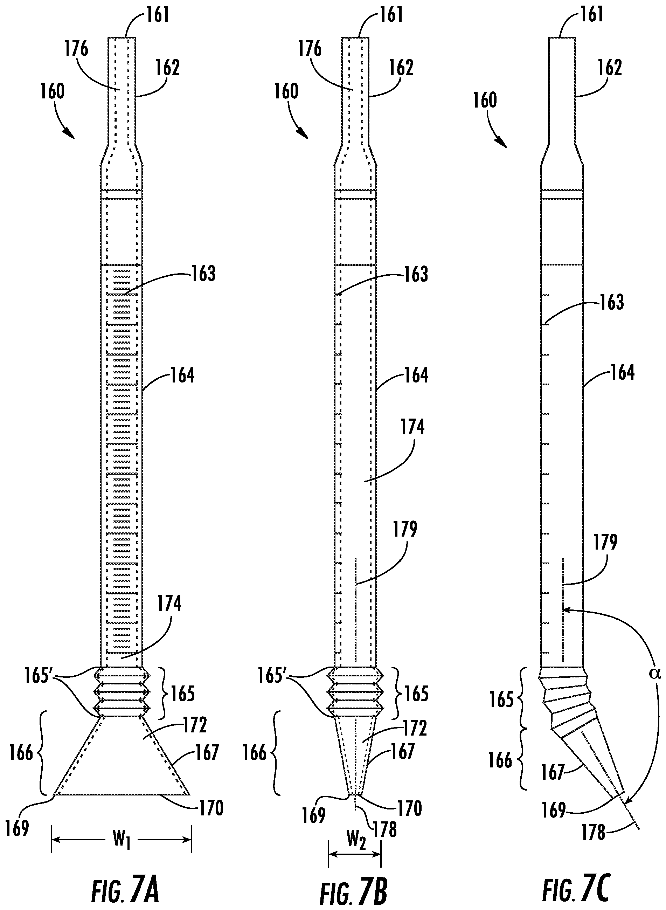

The pipettes include a bellows joint permitting pivotal movement between first and second body sections thereof. FIGS. 7A-7C illustrate a serological pipette 160 similar to the pipette 100 of FIGS. 4A-4C, but including a bellows joint 165 instead of a non-linear elbow transition between a first body section 164 and a second body section 166. The pipette 160 includes a mouthpiece 162 proximate to a mouth end 161, a first body section 164 having a series of graduated markings 163, and a second body section 166 that is proximate to a sample introduction end 169. A bellows joint 165 is provided between the first and second body sections 164, 166. Optionally, welded interfaces 165' may be provided between the bellows joint 165 and the first and second body sections 164, 166. The second body section 166 includes a wall structure 167 bounding a fluid passage 172 (i.e., a second fluid passage) that opens to a sample introduction port 170 at the sample introduction end 169. Corresponding fluid passages 174, 176 are defined in the first body section 164 and the mouthpiece 162. The second body section 166 has a non-uniform primary width (along primary width dimension W₁) that increases with proximity to the sample introduction end 169 and that is greater than that of the first body section 164, and the second fluid passage 172 has a primary width that increases with proximity to the sample introduction end 169 and that is greater than that of the first fluid passage 174. The second body section 166 has a non-uniform transverse width (along transverse width dimension W₂) that decreases with proximity to the sample introduction end 169 and that is smaller than that of the first body section 164, and the second fluid passage 172 has a transverse width that decreases with proximity to the sample introduction end 169 and that is smaller than that of the first fluid passage 174. FIGS. 7A and 7B provide front elevational and right side elevational views of the pipette 160 with the bellows joint 165 in a straight configuration arranged between the first and second body sections 164, 166, with central longitudinal axes 179, 178 of first and second body sections 164, 166 being collinearly arranged. FIG. 7C is a right side elevational view of the pipette 160, following pivotal movement of the bellows joint 165 to cause central longitudinal axes 179, 178 of the first and second body sections 164, 167 to be non-parallel, with such axes 179, 178 being oriented at an angle α relative to one another. The angle α may be adjusted to any range previously described herein. Presence of the bellows joint 165 permits the angle α to be adjusted as desired by a user. The bellows joint 165 may be adjusted (e.g., by a user) to a certain position and then maintain that position until intentionally re-adjusted. Permitting angular variation between the first and second body sections 164, 167 of the pipette 160 may be useful, for example, to target different microwell regions along a structured surface of a cell culture housing when transferring material between an interior of the cell culture housing and an external environment. Alternatively, such angular variation may be useful to either position a sample introduction end away from microwells to preferentially remove cell culture medium from the interior of a cell culture housing during a material extraction step, or to position a sample introduction end proximate to one or more selected microwells to preferentially remove at least some multi-cell aggregates during a material extraction step.

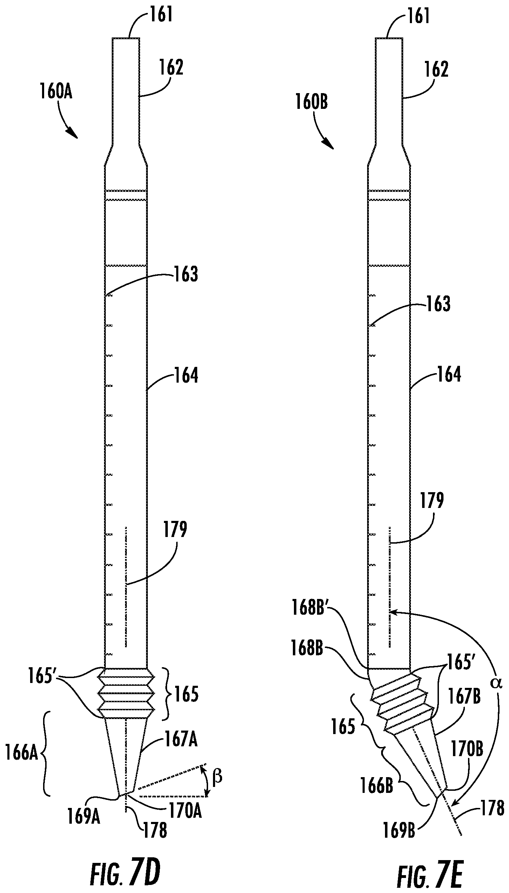

A pipette including a bellows joint may include a tip of a sample introduction end that is offcut at an angle non-perpendicular to a central longitudinal axis of a second body portion. FIG. 7D illustrates an alternative serological pipette 160A similar to the pipette 160 of FIGS. 7A-7C, with a tip of the sample introduction end 169A being offcut at an angle non-perpendicular to a central longitudinal axis 178 of the second body section 166A, and with the bellows joint 165 in a straight configuration arranged between the first and second body sections 164, 166A. Welded interfaces 165' optionally may be provided between the bellows joint 165 and the first and second body sections 164, 166A. The pipette 160A includes a mouthpiece 162 proximate to a mouth end 161, the first body section 164 having a series of graduated markings 163, and the second body section 166A that is proximate to a sample introduction end 169A. The second body section 166A includes a wall structure 167A bounding a fluid passage that opens to a sample introduction port 170A at the sample introduction end 169A. Fluid passages are defined in the pipette 160A in the same manner as the pipette 160 of FIGS. 7A-7C. The first body section 164 has a central longitudinal axis 179 that is parallel to the central longitudinal axis 178 of the second body section 166A. The tip of the sample introduction end 169A is offcut at an angle β that is non-perpendicular to the central longitudinal axis 178 of the second body section 166A (which as illustrated is collinear with a central longitudinal axis 179 of the first body section 164. In certain embodiments, the tip of the sample introduction end 169A is offcut at an angle in a range of from 5 degrees to 45 degrees (or any other range disclosed herein for an offcut tip).

In certain embodiments, a pipette including a bellows joint may additionally include a non-linear elbow transition arranged between first and second body sections. FIG. 7E illustrates an alternative serological pipette 160B similar to the pipette 160A, with addition of a non-linear elbow transition 168B arranged between a first body section 164 and a bellows joint 165, and with the bellows joint 165 (in a straight configuration) being arranged between the non-linear elbow transition 168B and the second body second 166B. Optionally, welded interfaces 165' may be provided between the bellows joint 165 and the second body section 166B, and between the bellows joint 165 and the non-linear elbow transition 168B. The pipette 160B includes a mouthpiece 162 proximate to a mouth end 161, the first body section 164 having a series of graduated markings 163, and the second body section 166B that is proximate to a sample introduction end 169B. The second body section 166B includes a wall structure 167B bounding a fluid passage that opens to a sample introduction port 170B at the sample introduction end 169B. Fluid passages are defined in the pipette 160B in the same manner as the pipette 160 of FIGS. 7A-7C. The first body section 164 has a central longitudinal axis 179 that is non-parallel to a central longitudinal axis 178 of the second body section 166B (e.g., due to presence of the non-linear elbow transition 168B), and such angle may be further adjusted as desired by manipulation of the bellows joint 165. A tip of the sample introduction end 169B is offcut at an angle β that is non-perpendicular to the central longitudinal axis 178 of the second body section 166B.

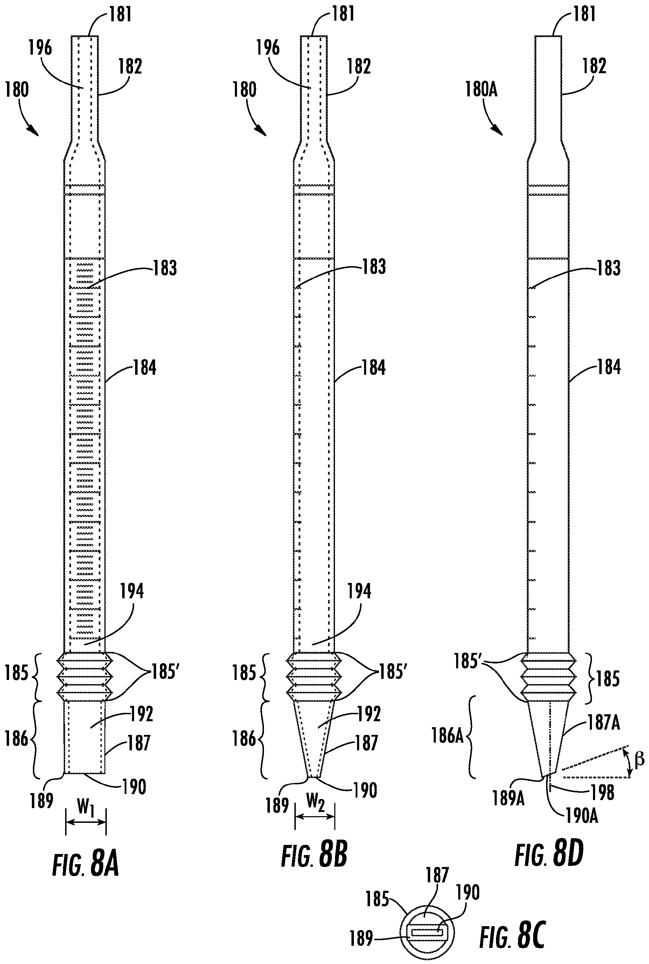

In certain embodiments, a pipette including a bellows joint may include a second body section having a width that is constant and/or reduced in one or more dimensions relative to a first body section. FIGS. 8A-8C illustrate a serological pipette 180 including a second body section 186 having a constant primary width and a reduced transverse width both relative to a first tubular body section 184, with a bellows joint 185 in a straight configuration arranged between the first and second body sections 184, 186. The bellows joint 185 is illustrated in a straight configuration arranged between the first and second body sections 184, 186. The pipette 180 includes a mouthpiece 182 proximate to a mouth end 181, the first body section 184 having a series of graduated markings 183, and the second body section 186 that is proximate to a sample introduction end 189. Optionally, welded interfaces 185' may be provided between the bellows joint and the first and second body sections 184, 186. The second body section 186 includes a wall structure 187 bounding a fluid passage 192 that opens to a sample introduction port 190 at the sample introduction end 189. Corresponding fluid passages 194, 196 are defined in the first body section 184 and the mouthpiece 182. The second body section 186 has a constant primary width (along primary width dimension W₁) that is equal to that of the first body section 184, and the second fluid passage 192 has a constant primary width that is equal to that of the first fluid passage 194. The second body section 186 has a non-uniform transverse width (along transverse width dimension W₂) that decreases with proximity to the sample introduction end 189 and that is smaller than that of the first body section 184, and the second fluid passage 192 has a transverse width that decreases with proximity to the sample introduction end 189 and that is smaller than that of the first fluid passage 194.

In certain embodiments, a pipette including a bellows joint, and including a second body section having a width that is reduced in one or more dimensions relative to a first body section, may further include a tip of a sample introduction end that is offcut at an angle non-perpendicular to a central longitudinal axis of a second body portion. FIG. 8D illustrates an alternative serological pipette 180A similar to the pipette 180 of FIGS. 8A-8C, with a tip of the sample introduction end 189A being offcut at an angle non-perpendicular to a central longitudinal axis 198 of the second body section 186A, and with the bellows joint 185 in a straight configuration arranged between the first and second body sections 184, 186A. The pipette 180A includes a mouthpiece 182 proximate to a mouth end 181, the first body section 184 with a series of graduated markings 183, and the second body section 186A that is proximate to a sample introduction end 189A. Optionally, welded interfaces 185' may be provided between the bellows joint 185 and the first and second body sections 184, 186A. The second body section 186A includes a wall structure 187A bounding a fluid passage that opens to a sample introduction port 190A at the sample introduction end 189A. Fluid passages are defined in the pipette 180A in the same manner as the pipette 180 of FIGS. 8A-8C. The tip of the sample introduction end 189A is offcut at an angle β that is non-perpendicular to the central longitudinal axis 198 of the second body section 186A. In certain embodiments, the tip of the sample introduction end 189A is offcut at an angle in a range of from 5 degrees to 45 degrees (or any other range disclosed herein for an offcut tip).

While various preceding embodiments have been directed to pipette structures in the form of measuring (e.g., serological) pipettes, in certain embodiments, pipette structures may be embodied in extension tip devices for use with conventional measuring pipettes. In such embodiments, a first body section defines a cavity configured to receive a tip region of a measuring pipette, and a second body section includes characteristics as defined herein. In certain embodiments, the cavity is bounded by an inner surface, and the inner surface defines an annular recess configured to receive a sealing ring to promote sealing and/or retention between the extension tip device and the tip region of the measuring pipette.

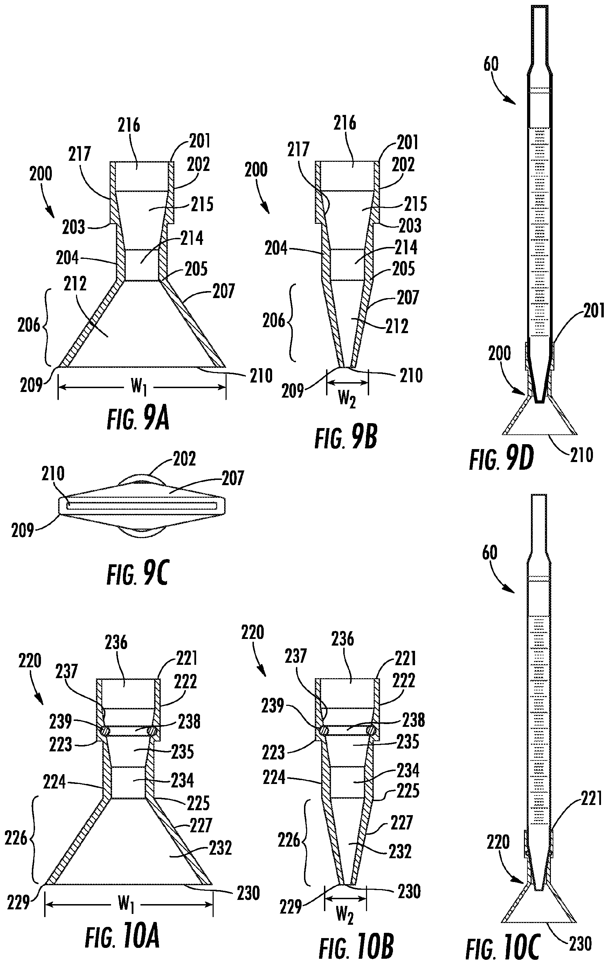

In certain embodiments, an extension tip device for a measuring pipette includes a fluid passage of a second body section having a greater width dimension at a sample introduction end than a width dimension of a first body section at the junction between the first and second body sections. FIGS. 9A-9C show an extension tip device 200 including an upper body section 202 configured to receive a tip region of a measuring pipette, a first body section 204 aligned with the upper body section 202, and a second body section 206 having an increased primary width (and a reduced transverse width) relative to the first body section 204. At least one junction 205 (optionally including a welded interface) is formed between the first and second body sections 204, 206. A shoulder 203 representing a change in outer diameter may be provided between the first and upper body sections 204, 202. The extension tip device 200 includes a pipette receiving end 201 defining a cavity 216 originating in the upper body section 202 that is bounded in part by an inwardly tapered surface 217 configured to sealingly engage an outer wall of a pipette (not shown) proximate to a tip thereof. The inwardly tapered surface 217 may continue into the first body section 204. The second body section 206 includes a wall structure 207 bounding a fluid passage 212 that opens to a sample introduction port 210 at the sample introduction end 209. Consecutively arranged fluid passages 214, 215 are defined in the first body section 204 and the upper body section 206, respectively, with the fluid passage 215 opening to the cavity 216 proximate to the pipette receiving end 201.

Various sections of the extension tip device 200 include a primary width dimension Wi (shown in FIG. 9A) and a transverse width dimension W₂ (shown in FIG. 9B) that may be orthogonal to the primary width dimension Wi. As shown in FIGS. 9A and 9B, the second body section 206 has a non-uniform primary width that increases with proximity to the sample introduction end 209 and that is greater than that of the first body section 204. Similarly, the second fluid passage 212 has a primary width that increases with proximity to the sample introduction end 209 and that is greater than that of the fluid passages 214, 215 defined in the first and upper body sections 204, 202. More specifically a maximum primary width of the second fluid passage 212 is greater than a maximum primary width of the first fluid passage 214 defined in the first body section 204. The second body section 206 and the second fluid passage 212 have non-uniform transverse widths that decrease with proximity to the sample introduction end 209, with the transverse widths being smaller than those of the corresponding first body section 204 and the first fluid passage 214. FIG. 9C shows a bottom view of the extension tip device of FIGS. 9A-9B, illustrating that the sample introduction port 210 has a much greater primary width relative to the transverse width.

FIG. 9D is a front, partial cross-sectional view of the extension tip device 200 of FIGS. 9A-9C, with a tip region of a measuring pipette 60 received in a cavity (i.e., cavity 216 shown in FIGS. 9A-9B) originating in the upper body section of the extension tip device 200 proximate to the pipette receiving end 201. An outer surface of the measuring pipette 60 may be sealingly engaged to an inwardly tapered surface (i.e., inwardly tapered surface 217 as shown in FIGS. 9A-9B) of the extension tip device 200. As shown, a fluid passage of the extension tip device 200 proximate to the sample introduction end 210 has a substantially greater width than fluid passages of the pipette 60. Engagement between the measuring pipette 60 and the extension tip device 200 permits the resulting assembly to function in a manner similar to the pipette 120 previously described in connection with FIGS. 5A-5D.

In certain embodiments, an extension tip device for a measuring pipette includes a sealing ring arranged in an annular recess to promote sealing and/or retention between an extension tip device and the tip region of a measuring pipette. FIGS. 10A and 10B show an extension tip device 220 that is similar to the extension tip device 200 described in connection with FIGS. 9A-9B, with the addition of a sealing ring (e.g., O-ring) 239 received within an annular recess 238 defined in an inwardly tapered surface 237. The extension tip device 220 includes an upper body section 222 configured to receive the tip region of a measuring pipette, a first body section 224 aligned with the upper body section 222, and a second body section 226 having an increased primary width (and a reduced transverse width) relative to the first body section 224. At least one junction 225 (optionally including a welded interface) is formed between the first and second body sections 224, 226. A shoulder 223 representing a change in outer diameter may be provided between the first and upper body sections 224, 222. A pipette receiving end 221 defines a cavity 236 originating in the upper body section 222 that is bounded in part by the inwardly tapered surface 237 configured to engage an outer wall of a pipette (not shown) proximate to the tip thereof, with the sealing ring 239 further assisting with engagement of the pipette. The inwardly tapered surface 237 may continue into the first body section 224. The second body section 226 includes a wall structure 227 bounding a fluid passage 232 that opens to a sample introduction port 230 at the sample introduction end 229. Consecutively arranged fluid passages 234, 235 are defined in the first body section 224 and the upper body section 226, respectively, with the fluid passage 235 opening to the cavity 236 proximate to the pipette receiving end 221. The fluid passages 232, 234, 235 defined in the extension tip device 220 are illustrated as having the same relative shapes and sizes as previously described for the corresponding passages 212, 214, 215 of the extension tip device 200 of FIGS. 8A-8C.

FIG. 10C is a front, partial cross-sectional view of the extension tip device 220 of FIGS. 10A-10B, with a tip region of a measuring pipette 60 received in a cavity (i.e., cavity 236 shown in FIGS. 10A-10B) originating in the upper body section of the extension tip device 220 proximate to the pipette receiving end 221. An outer surface of the measuring pipette 60 may be sealingly engaged to an inwardly tapered surface and sealing ring (i.e., inwardly tapered surface 237 and sealing ring 239 as shown in FIGS. 10A-10B) of the extension tip device 220. As shown, a fluid passage of the extension tip device 220 proximate to the sample introduction end 230 has a substantially greater width than fluid passages of the pipette 60. Engagement between the measuring pipette 60 and the extension tip device 220 permits the resulting assembly to function in a manner similar to the pipette 120 previously described in connection with FIGS. 5A-5D.

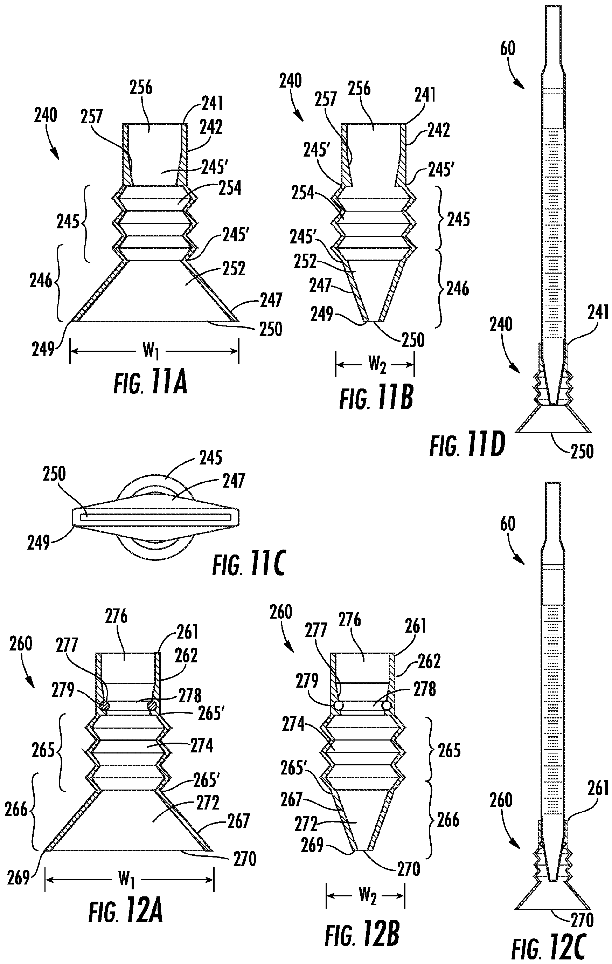

An extension tip device includes a bellows joint permitting pivotal movement between first and second body sections thereof. FIGS. 11A-11C illustrate an extension tip device 240 similar to the extension tip device 200 of FIGS. 9A-9C, including a first body section 242 configured to receive a tip region of a measuring pipette, and including a second body section 246 having an increased primary width (and a reduced transverse width) relative to the first body section 242, with a bellows joint 245 (illustrated in a straight configuration, but being capable of pivotal movement) between the first and second body sections 242, 246. Optionally, welded interfaces 245' may be provided between the bellows joint 245 and the first and second body sections 242, 246. The extension tip device 240 includes a pipette receiving end 241 defining a cavity (also embodying a fluid passage) 256 originating in the first body section 242 that is bounded by an inwardly tapered surface 257 configured to sealingly engage an outer wall of a pipette (not shown) proximate to a tip thereof. The second body section 246 includes a wall structure 247 bounding a fluid passage 252 that opens to a sample introduction port 250 at the sample introduction end 249. The bellows joint 245 defines a fluid passage 254 that extends between the fluid passages 256, 252 defined in the first and second body sections 244, 246, with the fluid passage 256 also serving as a cavity 256 proximate to the pipette receiving end 241. Various sections of the extension tip device 240 include a primary width dimension Wi (shown in FIG. 11A) and a transverse width dimension W₂ (shown in FIG. 11B) that may be orthogonal to the primary width dimension W₁. As shown, the second body section 246 has a non-uniform primary width that increases with proximity to the sample introduction end 249 and that is greater than that of the first body section 242. Similarly, the second fluid passage 252 has a primary width that increases with proximity to the sample introduction end 249 and that is greater than that of the fluid passage 256 defined in the first body section 242. The second body section 246 and the second fluid passage 242 have non-uniform transverse widths that decrease with proximity to the sample introduction end 249, with the transverse widths being smaller than those of the corresponding first body section 242 and the first fluid passage 256. FIG. 11C shows a bottom view of the extension tip device 240 of FIGS. 11A-11B, illustrating that the sample introduction port 250 has a much greater primary width relative to the transverse width.

FIG. 11D is a front, partial cross-sectional view of the extension tip device 240 of FIGS. 11A-11C, with a tip region of a measuring pipette 60 received in a cavity (i.e., cavity 246 shown in FIGS. 11A-11B) defined in the upper body section of the extension tip device 240. An outer surface of the measuring pipette 60 may be sealingly engaged to an inwardly tapered surface (i.e., inwardly tapered surface 257 as shown in FIGS. 11A-11B) of the extension tip device 240. As shown, a fluid passage of the extension tip device 240 proximate to the sample introduction end 250 has a substantially greater width than fluid passages of the pipette 60. Engagement between the measuring pipette 60 and the extension tip device 240 permits the resulting assembly to function in a manner similar to the pipette 160 previously described in connection with FIGS. 7A-7C.

An extension tip device includes a bellows joint permitting pivotal movement between first and second body sections thereof, and may further include a sealing ring arranged in an annular recess to promote sealing and/or retention between the extension tip device and the tip region of a measuring pipette. FIGS. 12A and 12B show an extension tip device 260 that is similar to the extension tip device 240 described in connection with FIGS. 11A-11B, with addition of a sealing ring (e.g., O-ring) 279 received within an annular recess 278 defined in an inwardly tapered surface 277. The extension tip device 260 includes first and second body sections 262, 266 with a bellows joint 265 arranged therebetween. Optionally, welded interfaces 265' may be provided between the bellows joint 265 and the first and second body sections 262, 266. The extension tip device 260 includes a pipette receiving end 261 defining a cavity (also embodying a fluid passage) 276 originating in the first body section 262 that is bounded by the inwardly tapered surface 277 configured to sealingly engage an outer wall of a pipette (not shown) proximate to a tip thereof. The second body section 266 includes a wall structure 267 bounding a fluid passage 272 that opens to a sample introduction port 270 at the sample introduction end 269. The bellows joint 265 defines a fluid passage 274 that extends between the fluid passages 276, 272 defined in the first and second body sections 262, 266, with the fluid passage 276 also serving as a cavity 276 proximate to the pipette receiving end 261. The fluid passages 272, 276 defined in the extension tip device 260 are illustrated as having the same relative shapes and sizes as previously described for the corresponding passages 252, 256 of the extension tip device 240 of FIGS. 11A-11C.

FIG. 12C is a front, partial cross-sectional view of the extension tip device 260 of FIGS. 11A-11B, with a tip region of a measuring pipette 60 received in a cavity (i.e., cavity 266 shown in FIGS. 11A-11B) defined in the first body section of the extension tip device 260 proximate to the pipette receiving end 261. An outer surface of the measuring pipette 60 may be sealingly engaged to an inwardly tapered surface and sealing ring (i.e., inwardly tapered surface 277 and sealing ring 279 as shown in FIGS. 12A-12B) of the extension tip device 260. As shown, a fluid passage of the extension tip device 260 proximate to the sample introduction port 270 has a substantially greater width than fluid passages of the pipette 60. Engagement between the measuring pipette 60 and the extension tip device 260 permits the resulting assembly to function in a manner similar to the pipette 120 previously described in connection with FIGS. 5A-5D.

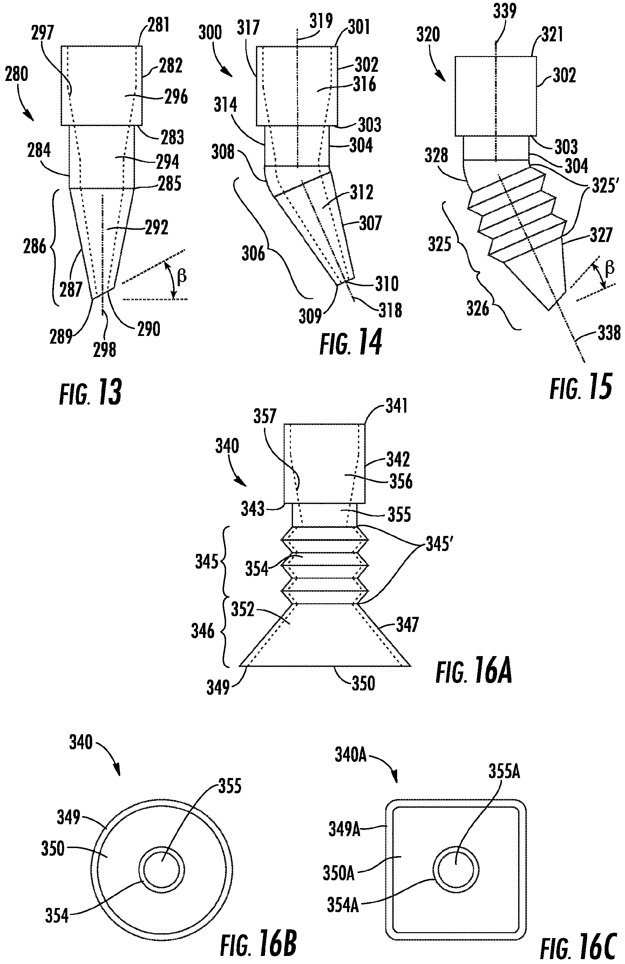

In certain embodiments, an extension tip device as described herein may include a tip of a sample introduction end that is offcut at an angle non-perpendicular to a central longitudinal axis of a second body portion. FIG. 13 is a right side elevational view of an extension tip device 280 similar to the extension tip device 200 illustrated in FIGS. 9A-9C, with a tip of the sample introduction end 289 being offcut at an angle β that is non-perpendicular to a central longitudinal axis 298 of a second body section 286. The extension tip device 280 includes an upper body section 282 configured to receive a tip region of a measuring pipette, the first body section 284 aligned with the upper body section 282, and the second body section 286 having an increased primary width (and a reduced transverse width) relative to the first body section 284. At least one junction 285 (optionally including a welded interface) is formed between the first and second body sections 284, 286. A shoulder 283 representing a change in outer diameter may be provided between the first and upper body sections 284, 282. The extension tip device 280 includes a pipette receiving end 281 defining a cavity 296 originating in the upper body section 282 that is bounded in part by an inwardly tapered surface 297 configured to sealingly engage an outer wall of a pipette (not shown) proximate to a tip thereof. The inwardly tapered surface 297 may continue into the first body section 284. The second body section 286 includes a wall structure 287 bounding a fluid passage 292 that opens to a sample introduction port 290 at the sample introduction end 289. Consecutively arranged fluid passages 294, 296 are defined in the first body section 284 and the upper body section 282, respectively, with the fluid passage 294 opening to the cavity 296 proximate to the pipette receiving end 281. The fluid passages 292, 294, 295 defined in the extension tip device 280 are illustrated as having the same relative shapes and sizes as previously described for the corresponding fluid passages 212, 214, 215 of the extension tip device 200 of FIGS. 9A-9C. In certain arrangements, the tip of the sample introduction end 289 is offcut at an angle in a range of from 5 degrees to 45 degrees (or any other range disclosed herein for an offcut tip).

An extension tip device as described herein includes a non-linear elbow transition arranged between first and second body sections. FIG. 14 is a right side elevational view of an extension tip device 300 similar to the extension tip device 200 illustrated in FIGS. 9A-9C, with addition of a non-linear elbow transition 308 arranged between a first body section 304 and a second body section 306. The first body section 304 has a central longitudinal axis 319 that is non-parallel to a central longitudinal axis 318 of the second body section 306 due to presence of the non-linear elbow transition 308. A shoulder 303 representing a change in outer diameter may be provided between the first and upper body sections 304, 302. The extension tip device 300 includes a pipette receiving end 301 defining a cavity 316 originating in the upper body section 302 that is bounded in part by an inwardly tapered surface 317 configured to sealingly engage an outer wall of a pipette (not shown) proximate to a tip thereof. The inwardly tapered surface 317 may continue into the first body section 304. The second body section 306 includes a wall structure 307 bounding a fluid passage 312 that opens to a sample introduction port 310 at the sample introduction end 309. A fluid passage 314 in the first body section 304 is in fluid communication with the fluid passage 312 and the cavity 316. The fluid passages 312, 314, 315 defined in the extension tip device 300 may have the same relative shapes and sizes as previously described for the corresponding passages 212, 214, 215 of the extension tip device 200 of FIGS. 9A-9C.

In certain embodiments, an extension tip device as described herein may include a non-linear elbow transition and a bellows joint arranged between first and second body sections, and a tip of a sample introduction end that is offcut at an angle non-perpendicular to a central longitudinal axis of a second body portion. FIG. 15 is a right side elevational view of an extension tip device 320 according to one embodiment similar to the extension tip device 240 of FIGS. 11A-11C, with a non-linear elbow transition 328 and a bellows joint 325 arranged between first and second body sections 304, 327. The first body section 304 is arranged between an upper body section 302 and the non-linear elbow transition 328. Optionally, welded interfaces 325' may be provided between the bellows joint 325 and the second body sections 326, and between the bellows joint 325 and the non-linear elbow transition 328. The upper body section 302 includes a pipette receiving end 321 defining a cavity suitable for receiving the end of a pipette. The first body section 304 has a central longitudinal axis 339 that is non-parallel to a central longitudinal axis 338 of the second body section 326 due to presence of the non-linear elbow transition 328. A shoulder 303 representing a change in outer diameter may be provided between the first and upper body sections 304, 302. The second body section 326 includes a sample introduction end 309 that is offcut at an angle β non-perpendicular to the central longitudinal axis 338 of the second body portion 326.

In certain embodiments, an extension tip device as described herein includes a bellows joint arranged between first and second body sections, and the sample introduction end may include a greater primary and transverse width dimensions than corresponding width dimensions of a first body section. FIGS. 16A and 16B provide front elevational and bottom views of an extension tip device 340 including a first body section 342 configured to receive a tip region of a measuring pipette, and including a second body section 346 having increased primary and transverse widths relative to the first body section 342. A bellows joint 345 (illustrated in a straight configuration, but being capable of pivotal movement) is provided between the first and second body sections 342, 346. Optionally, welded interfaces 345' may be provided between the bellows joint 345 and the first and second body sections 342, 346. The extension tip device 340 includes a pipette receiving end 341 through which a cavity (also embodying a fluid passage) 356 extends into the first body section 342, with the cavity 356 being bounded by an inwardly tapered surface 357 configured to sealingly engage an outer wall of a pipette (not shown) proximate to a tip thereof. A shoulder 343 is provided along a portion of the first body section 342, corresponding to a reduced width portion of the first body section 342 that bounds a fluid passage 355. The second body section 346 includes a wall structure 347 bounding a fluid passage 352 that opens to a sample introduction port 350 at the sample introduction end 349. The bellows joint 345 also defines a fluid passage 354. From the sample introduction end 349 toward the pipette receiving end 341, the fluid passages 352, 354, 355, 356 are consecutively arranged and in fluid communication with one another. As shown, the second body section 346 and the second fluid passage 352 have non-uniform primary and transverse widths that increase with proximity to the sample introduction end 349, with such widths being greater than the corresponding primary and transverse widths of the first body section 342 and each fluid passage 355, 356 of the first body section 342, respectively. As shown in FIGS. 16A-16B, the wall structure 347 and a fluid passage 352 may be frustoconical in shape with a round cross-section. It is noted that a wall structure and fluid passage may have any suitable cross-sectional shape. For example, FIG. 16C is a bottom plan view of an alternative extension tip device 340A similar to the extension tip of FIGS. 16A-16B, illustrating a sample introduction end 349A and a sample introduction port 350A as being substantially square in shape, in combination with fluid passages 354A, 355A both having round cross-sectional shapes. Engagement between a measuring pipette and the extension tip device 340 permits the resulting assembly to function in a manner similar to the pipette 140 previously described in connection with FIGS. 6A-6D.

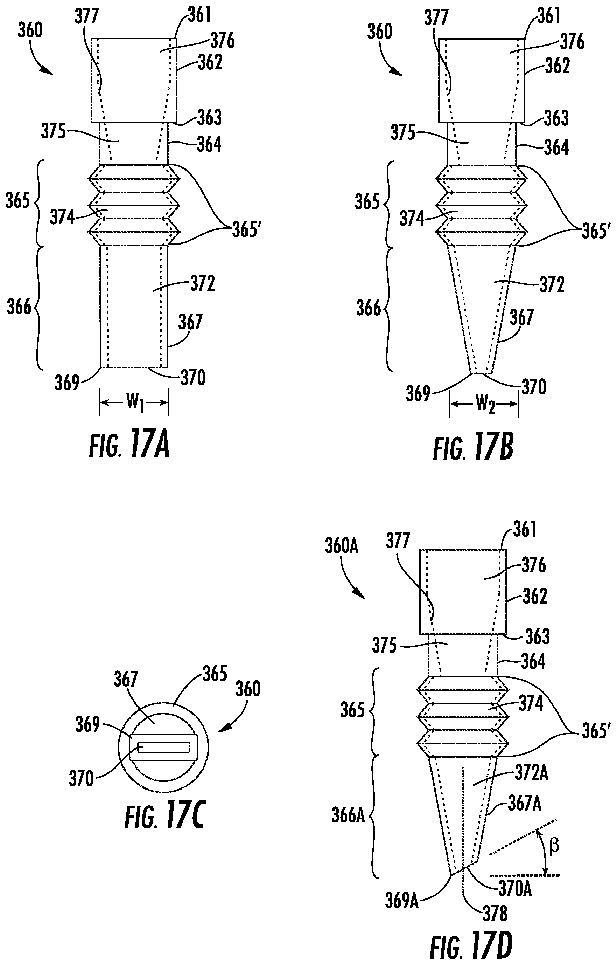

In certain embodiments, an extension tip device having a bellows joint may include a second body section having a width that is constant and/or reduced in one or more dimensions relative to a first body section. FIGS. 17A-17C illustrate an extension tip device 360 including an upper body section 362 configured to receive a tip region of a measuring pipette, a first body section 364 aligned with the upper body section 362, and a second body section 366 having an increased primary width (and a reduced transverse width) relative to the first body section 364. A bellows joint 365 in a straight configuration is arranged between the first and second body sections 364, 366. Optionally, welded interfaces 365' may be provided between the bellows joint 365 and the first and second body sections 364, 366A. A shoulder 363 representing a change in outer diameter may be provided between the first and upper body sections 364, 362. The second body section 366 includes a wall structure 367 bounding a fluid passage 372 that opens to a sample introduction port 370 at the sample introduction end 369. Corresponding fluid passages 374, 375, 377 are defined in the bellows joint 365, the first body section 364, and the upper body section 362. The fluid passages 377, 375 defined in the first body section 364 and the upper body section 362, respectively, are bounded by a tapered wall and have a width that decreases with proximity to the bellows joint 365. The second body section 366 has a constant primary width (along primary width dimension W₁) that is equal to that of the first body section 364, and the second fluid passage 372 has a constant primary width. The second body section 366 has a non-uniform transverse width (along transverse width dimension W₂) that decreases with proximity to the sample introduction end 369 and that is smaller than that of the first body section 364, and the second fluid passage 372 has a transverse width that decreases with proximity to the sample introduction end 369 and that is smaller than that of the first fluid passage 375. As shown in FIG. 17C, the sample introduction port 370 of the extension tip device 360 has a substantially greater primary width than transverse width. Engagement between a measuring pipette and the extension tip device 360 permits the resulting assembly to function in a manner similar to the pipette 180 previously described in connection with FIGS. 8A-8C.

An extension tip device similar to the extension tip device 360 of FIGS. 17A-17C may include an offcut tip. FIG. 17D is a right side elevational view of an extension tip device 360A similar to the extension tip device 360 illustrated in FIGS. 17A-17C, with a tip of the sample introduction end 369A being offcut at an angle β that is non-perpendicular to a central longitudinal axis 378 of a second body section 366A. The extension tip device includes an upper body section 362 configured to receive a tip region of a measuring pipette, a first body section 364 aligned with the upper body section 362, and a second body section 366A having an increased primary width (and a reduced transverse width) relative to the first body section 364. A bellows joint 365 in a straight configuration is arranged between the first and second body sections 364, 366. Optionally, welded interfaces 365' may be provided between the bellows joint 365 and the first and second body sections 364, 366. A shoulder 363 representing a change in outer diameter may be provided between the first and upper body sections 364, 362. The second body section 366A includes a wall structure 367A bounding a fluid passage 372A that opens to a sample introduction port 370A at the sample introduction end 369A. Corresponding fluid passages 374, 375, 377 are defined in the bellows joint 365, the first body section 364, and the upper body section 362. Engagement between a measuring pipette and the extension tip device depicted in FIG. 17D permits the resulting assembly to function in a manner similar to the pipette 180A previously described in connection with FIG. 8D.

As noted previously, pipette structures disclosed herein are suitable for transferring material between an interior of a cell culture housing (such as the housings disclosed in FIGS. 2A and 3). The capability of certain pipette structures disclosed herein to permit an angle between first and second body sections to be adjusted (e.g., using a bellows joint) enables a user to preferentially remove multi-cell aggregates or preferentially remove cell culture medium from such a housing.

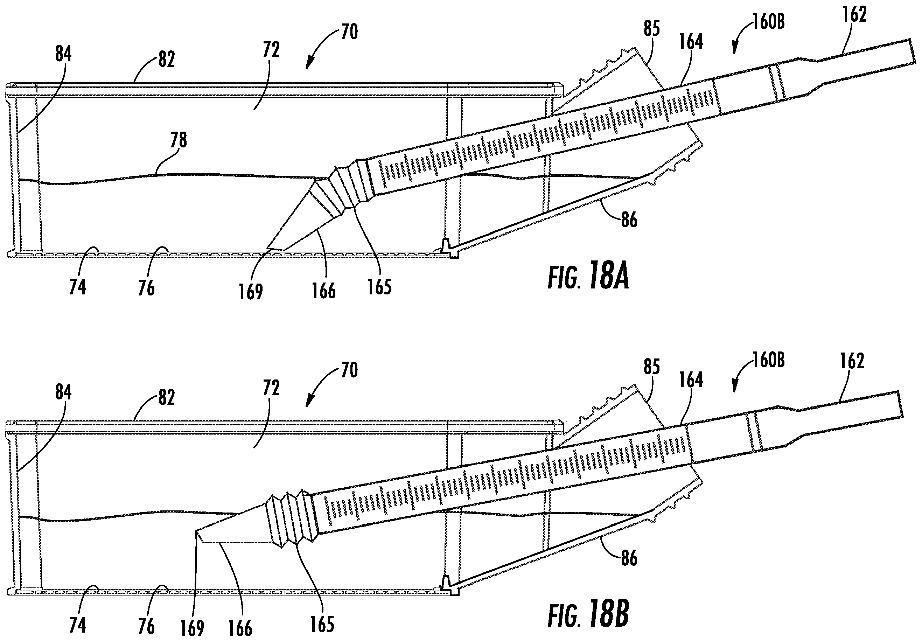

For example, FIG. 18A illustrates a cell culture housing 70 receiving therein a portion of a serological pipette 160B, with the pipette 160B including a bellows joint 166 permitting pivotal movement between first and section body sections 164, 166 of the pipette 160B. As shown, the bellows joint 166 is arranged in a non-linear position, with the first and second body sections 164, 166 attached to the bellows joint 166 being non-parallel to one another. The cell culture housing 70 encloses a cell culture chamber 72, with the bottom of the cell culture chamber 72 containing a structured surface 74 defining an array of microwells 76. A cell culture medium 78 is arranged in the cell culture chamber 72 above the structured surface 74. As shown in FIG. 18A, the sample introduction end 169 of the pipette 160B is offcut and positioned proximate to microwells 76 (e.g., optionally contacting the structured surface 74 defining the microwells 76) in a manner to preferentially remove multi-cell aggregates from one or more selected microwells 76. The cell culture housing 70 further includes a top wall 82, side walls 84 extending upward from the structured surface 74 to the top wall 82, and a tubular neck 86 that extends outward and upward to define a port opening 85. Upon establishment of a subatmospheric pressure in the mouthpiece 162, contents of the cell culture housing 70 proximate to the sample introduction end 169 may be removed from the cell culture chamber 72 through the pipette 160B. Since the sample introduction end 169 of FIG. 18A is positioned on or proximate to the structured surface 74, the pipette 160B is positioned to preferentially remove multi-cell aggregates (relative to cell culture medium) from the cell culture chamber 72.

FIG. 18B illustrates the same cell culture housing 70 and serological pipette 160B illustrated in FIG. 18A, with the first and second body sections 164, 167 being collinearly arranged, and with the sample introduction end 169 of the pipette 160B being spaced apart from the structured surface 74 of the cell culture housing 70. Such positioning is configured to preferentially remove cell culture medium 78 from the interior of the cell culture housing 70 while reducing a likelihood of removing cell multi-cell aggregates from the array of microwells 76.

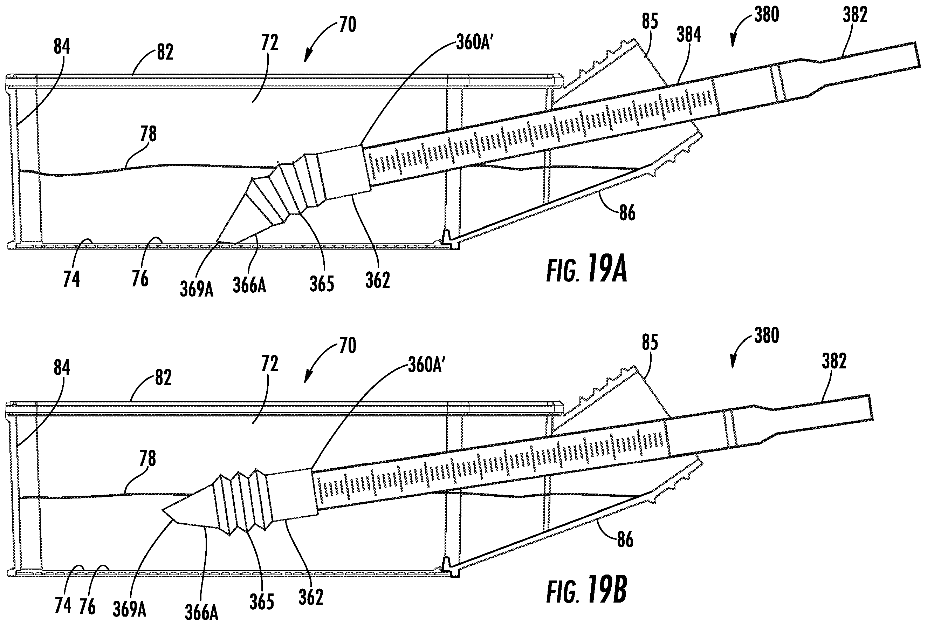

FIGS. 19A and 19B illustrate similar subject matter as FIGS. 18A and 18B, but each utilizing a pipette structure in the form of an extension tip device 360A' (similar to the structure shown in FIG. 17D) in combination with a conventional measuring pipette (instead of using the pipette 160B of FIGS. 18A and 18B). FIG. 19A illustrates a cell culture housing 70 receiving therein a portion of a pipette 380 having coupled thereto an extension tip device 360A' having first and second body sections 362, 366A and a bellows joint 365 arranged in a non-linear position, with the first and second body sections 362, 366A attached to the bellows joint 365 being non-parallel to one another. The cell culture housing 70 encloses a cell culture chamber 72, with the bottom of the cell culture chamber 72 containing a structured surface 74 defining an array of microwells 76, and a cell culture medium 78 is arranged in the cell culture chamber 72. As shown in FIG. 19A, the sample introduction end 369A of the extension tip device 360A' is offcut and positioned proximate to microwells 76 in a manner to preferentially remove multi-cell aggregates from one or more selected microwells 76. The cell culture housing 70 further includes a top wall 82, side walls 84, and a tubular neck 86 that defines a port opening 85. Upon establishment of a subatmospheric pressure in the mouthpiece 382 of the pipette 380, contents of the cell culture housing 70 proximate to the sample introduction end 369A may be removed from the cell culture chamber 72 through the extension tip device 360A' and the pipette 380. Since the sample introduction end 369A of FIG. 19A is positioned on or proximate to the structured surface 74, the extension tip device 360A' in combination with the pipette 380 are is positioned to preferentially remove multi-cell aggregates (relative to cell culture medium) from the cell culture chamber 72.

FIG. 19B illustrates the same cell culture housing 70, serological pipette 380, and extension tip device 360A' illustrated in FIG. 18A, with the first and second body sections 362, 366A being collinearly arranged, and with the sample introduction end 369A of the extension tip device 360A' being spaced apart from the structured surface 74 of the cell culture housing 70. Such positioning is configured to preferentially remove cell culture medium 78 from the interior of the cell culture housing 70 while reducing a likelihood of removing cell multi-cell aggregates from the array of microwells 76.

In further aspects of the disclosure, it is specifically contemplated that any two or more aspects, embodiments, or features disclosed herein may be combined for additional advantage.

As used herein, the singular forms "a," "an" and "the" include plural referents unless the context clearly dictates otherwise.

The term "include" or "includes" means encompassing but not limited to, that is, inclusive and not exclusive.

"Optional" or "optionally" means that the subsequently described event, circumstance, or component, can or cannot occur, and that the description includes instances where the event, circumstance, or component, occurs and instances where it does not.

Ranges can be expressed herein as from "about" one particular value, and/or to "about" another particular value. When such a range is expressed, examples include from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that any particular order be inferred. Any recited single or multiple feature or aspect in any one claim can be combined or permuted with any other recited feature or aspect in any other claim or claims.

It is also noted that recitations herein refer to a component being "configured" or "adapted to" function in a particular way. In this respect, such a component is "configured" or "adapted to" embody a particular property, or function in a particular manner, where such recitations are structural recitations as opposed to recitations of intended use. More specifically, the references herein to the manner in which a component is "configured" or "adapted to" denotes an existing physical condition of the component and, as such, is to be taken as a definite recitation of the structural characteristics of the component.

While various features, elements or steps of particular embodiments may be disclosed using the transitional phrase "comprising," it is to be understood that alternative embodiments, including those that may be described using the transitional phrases "consisting" or "consisting essentially of," are implied.

It will be apparent to those skilled in the art that various modifications and variations can be made to the present inventive technology without departing from the scope of the disclosure. Since modifications, combinations, sub-combinations and variations of the disclosed embodiments incorporating the substance of the inventive technology may occur to persons skilled in the art, the inventive technology should be construed to include everything within the scope of the appended claims.

## Claims

1. A pipette structure (160, 160B, 180, 240, 260, 320, 340, 360, 360A') comprising:
a first body section (164, 184, 242, 362) defining a first fluid passage (174, 194, 256);
a second body section (166, 186, 246, 366A) comprising a sample introduction end, being connected to the first body section (164, 184, 242, 362) at a junction, and defining a second fluid passage (172, 192, 252) in fluid communication with the first fluid passage (174, 194, 256);
wherein the pipette structure comprises features (i) and (ii):
(i) the junction comprises a bellows joint (165, 185, 245, 365) permitting pivotal movement between the first body section (164, 184, 242, 362) and the second body section (166, 186, 246, 366A) and a non-linear elbow transition (168B, 328) arranged between the first body section (164, 304) and the second body section (166, 327), wherein the non-linear elbow transition (166, 328) comprises a transition angle in a range of from 5 degrees to 60 degrees; and
(ii) the second fluid passage (172, 192, 252) comprises a non-uniform width between the sample introduction end (169, 189, 249) and the junction, with a width dimension at the sample introduction end (169, 189, 249) that is greater than a width dimension at the junction.

2. The pipette structure of claim 1, embodied in a unitary measuring pipette (160, 180), wherein:
the first body section (164, 184) comprises a tubular body of the measuring pipette (160, 180);
the second body section (166, 186) comprises a tip region (169, 189) of the measuring pipette (160, 180); and
the tubular body is arranged between the tip region (169, 189) and a mouth region (161, 181) of the measuring pipette (160, 180).

3. The pipette structure of claim 1, embodied in an extension tip device (240, 260) for use with a measuring pipette (60), wherein the first body section (242) defines a cavity (256, 246, 276) configured to receive a tip region of the measuring pipette (60).

4. The pipette structure of claim 3, wherein:
the cavity (276) is bounded by an inner surface (277), and the inner surface (277) defines an annular recess (278) configured to receive a sealing ring (279) to promote sealing between the extension tip device (260) and the tip region of the measuring pipette (60);
at least a portion of the second body section (166A, 166B, 286) proximate to the sample introduction end (169, 189, 249) comprises a central longitudinal axis (178, 289), and a tip of the sample introduction end (169, 189, 249) is offcut at an angle (β) non-perpendicular to the central longitudinal axis (178, 289);
the tip of the sample introduction end (169, 189, 249) is offcut at an angle in a range of from 5 degrees to 45 degrees from perpendicular to the central longitudinal axis (178);
a tip of the sample introduction end (349) comprises a primary width dimension and a transverse width dimension, wherein the primary width dimension is at least two times greater than the transverse width dimension;
the primary width dimension at the sample introduction end (369) is larger than the width dimension at the junction, and the transverse width dimension at the sample introduction end (369) is smaller than the width dimension at the junction;
the first body section (164) and the second body section (166) comprise at least one polymeric material;
or a combination thereof.

5. The pipette structure of any of claims 1-4, further comprising a welded interface (165') between the first body section (164) and the second body section (166) along at least a portion of the junction.

6. The pipette structure of any of claims 1-5, wherein the first body section (164) and the second body section (166) are configured or are configurable to permit a central longitudinal axis (178) of the second body section (166) to be oriented at an angle in a range of from 100 degrees to 170 degrees relative to a central longitudinal axis (179) of the first body section (164).

7. A method for transferring at least one material between (i) an interior of a cell culture housing (70) that includes a structured surface (74) defining an array of microwells (76) suitable for three-dimensional culture of multi-cell aggregates and (ii) an environment external to the cell culture housing (70), the method comprising:
inserting at least a portion of a pipette structure (160B, 360A') through a port (85) of the cell culture housing (70) into the interior of the cell culture housing (70), wherein the pipette structure (160B, 360A') includes: a first body section (164, 362) defining a first fluid passage; a second body section (166, 366A) comprising a sample introduction end (169, 369A), being connected to the first body section (164, 362) at a junction, and defining a second fluid passage in fluid communication with the first fluid passage; and features (i) and (ii):
(i) the junction comprises a bellows joint (165, 365) permitting pivotal movement between the first body section (164, 362) and the second body section (166, 366A); and
(ii) the second fluid passage comprises a non-uniform width between the sample introduction end (169, 369A) and the junction, with a width dimension at the sample introduction end (169, 369A) that is greater than a width dimension at the junction;
effectuating pivotal movement of the bellows joint (165, 365) to adjust orientation between a central longitudinal axis of the second body section (166, 366A) and a central longitudinal axis of the first body section (164, 362);
extracting the at least one material from the interior of the cell culture housing into or through the pipette structure (160B, 360A'); and
withdrawing the at least a portion of the pipette structure (160B, 360A') from the interior of the cell culture housing (70).

8. The method of claim 7, wherein the at least one material comprises cell culture medium (78) within the interior of the cell culture housing (70), and the method further comprises maintaining the sample introduction end (169, 369A) in a non-contacting relationship relative to the structured surface (74) in a manner to preferentially remove cell culture medium (78) from the interior of the cell culture housing (70) while reducing a likelihood of removing cell multi-cell aggregates from the array of microwells (76) during said extracting of the at least one material from the interior of the cell culture housing (70).

9. The method of claim 7, wherein the at least one material comprises the multi-cell aggregates, and the method further comprises maintaining the sample introduction end proximate to one or more selected microwells (76) of the array of microwells (76) in a manner to preferentially remove at least some multi-cell aggregates from the one or more selected microwells (76) during said extracting of the at least one material from the interior of the cell culture housing (70).

10. A method for fabricating the pipette structure of claim 1, the method comprising:
supplying a heated parison to a mold;
creating a differential pressure between an interior and an exterior of the parison to cause the parison to expand and conform to a cavity of the mold;
opening the mold; and
ejecting the pipette structure from the mold.

## Patentansprüche

1. Pipettenstruktur (160, 160B, 180, 240, 260, 320, 340, 360, 360A'), umfassend:
einen ersten Körperabschnitt (164, 184, 242, 362), der einen ersten Fluiddurchgang (174, 194, 256) definiert;
einen zweiten Körperabschnitt (166, 186, 246, 366A), der ein Probeneinführungsende umfasst, mit dem ersten Körperabschnitt (164, 184, 242, 362) an einer Verbindung verbunden ist und einen zweiten Fluiddurchgang (172, 192, 252) in Fluidkommunikation mit dem ersten Fluiddurchgang (174, 194, 256) definiert;
wobei die Pipettenstruktur Merkmal (i) und (ii) umfasst:
(i) die Verbindung umfasst ein Faltenbalggelenk (165, 185, 245, 365), das Schwenkbewegung zwischen dem ersten Körperabschnitt (164, 184, 242, 362) und dem zweiten Körperabschnitt (166, 186, 246, 366A) ermöglicht, und einen nichtlinearen Ellbogenübergang (168B, 328), der zwischen dem ersten Körperabschnitt (164, 304) und dem zweiten Körperabschnitt (166, 327) angeordnet ist, wobei der nichtlineare Ellbogenübergang (166, 328) einen Übergangswinkel in einem Bereich von 5 Grad bis 60 Grad umfasst; und
(ii) der zweite Fluiddurchgang (172, 192, 252) umfasst eine ungleichmäßige Breite zwischen dem Probeneinführungsende (169, 189, 249) und der Verbindung, mit einer Breitenabmessung an dem Probeneinführungsende (169, 189, 249), die größer als eine Breitenabmessung an der Verbindung ist.

2. Pipettenstruktur nach Anspruch 1, verkörpert in einer einheitlichen Messpipette (160, 180), wobei:
der erste Körperabschnitt (164, 184) einen röhrenförmigen Körper der Messpipette (160, 180) umfasst;
der zweite Körperabschnitt (166, 186) einen Spitzenbereich (169, 189) der Messpipette (160, 180) umfasst; und
der röhrenförmige Körper zwischen dem Spitzenbereich (169, 189) und einem Mündungsbereich (161, 181) der Messpipette (160, 180) angeordnet ist.

3. Pipettenstruktur nach Anspruch 1, verkörpert in einer Verlängerungsspitzenvorrichtung (240, 260) zur Verwendung mit einer Messpipette (60), wobei der erste Körperabschnitt (242) einen Hohlraum (256, 246, 276) definiert, der konfiguriert ist, um einen Spitzenbereich der Messpipette (60) aufzunehmen.

4. Pipettenstruktur nach Anspruch 3, wobei:
der Hohlraum (276) durch eine Innenfläche (277) begrenzt ist und die Innenfläche (277) eine ringförmige Aussparung (278) definiert, die konfiguriert ist, um einen Dichtungsring (279) aufzunehmen, um Abdichtung zwischen der Verlängerungsspitzenvorrichtung (260) und dem Spitzenbereich der Messpipette (60) zu fördern;
mindestens ein Teil des zweiten Körperabschnittes (166A, 166B, 286) nahe dem Probeneinführungsende (169, 189, 249) eine zentrale Längsachse (178, 289) umfasst und eine Spitze des Probeneinführungsendes (169, 189, 249) in einem Winkel (β) abgeschnitten ist, der nicht senkrecht zu der zentralen Längsachse (178, 289) ist;
die Spitze des Probeneinführungsendes (169, 189, 249) in einem Winkel in einem Bereich von 5 Grad bis 45 Grad von senkrecht zu der zentralen Längsachse (178) abgeschnitten ist;
eine Spitze des Probeneinführungsendes (349) eine primäre Breitenabmessung und eine Querbreitenabmessung umfasst, wobei die primäre Breitenabmessung mindestens zweimal größer als die Querbreitenabmessung ist;
die primäre Breitenabmessung an dem Probeneinführungsende (369) größer als die Breitenabmessung an der Verbindung ist, und die Querbreitenabmessung an dem Probeneinführungsende (369) kleiner als die Breitenabmessung an der Verbindung ist;
der erste Körperabschnitt (164) und der zweite Körperabschnitt (166) mindestens ein Polymermaterial umfassen;
oder eine Kombination davon.

5. Pipettenstruktur nach einem der Ansprüche 1-4, ferner umfassend eine geschweißte Schnittstelle (165') zwischen dem ersten Körperabschnitt (164) und dem zweiten Körperabschnitt (166) entlang mindestens eines Teils der Verbindung.

6. Pipettenstruktur nach einem der Ansprüche 1-5, wobei der erste Körperabschnitt (164) und der zweite Körperabschnitt (166) konfiguriert oder konfigurierbar sind, um einer zentralen Längsachse (178) des zweiten Körperabschnittes (166) zu ermöglichen, in einem Winkel in einem Bereich von 100 Grad bis 170 Grad relativ zu einer zentralen Längsachse (179) des ersten Körperabschnittes (164) ausgerichtet zu sein.

7. Verfahren zum Übertragen von mindestens einem Material zwischen (i) einem Inneren eines Zellkulturgehäuses (70), das eine strukturierte Oberfläche (74) beinhaltet, die eine Reihe von Mikrovertiefungen (76) definiert, die für dreidimensionale Kultur von Mehrzellaggregaten geeignet sind, und (ii) einer Umgebung außerhalb des Zellkulturgehäuses (70), wobei das Verfahren Folgendes umfasst:
Einführen von mindestens einem Teil einer Pipettenstruktur (160B, 360A') durch eine Öffnung (85) des Zellkulturgehäuses (70) in das Innere des Zellkulturgehäuses (70), wobei die Pipettenstruktur (160B, 360A') Folgendes beinhaltet: einen ersten Körperabschnitt (164, 362), der einen ersten Fluiddurchgang definiert; einen zweiten Körperabschnitt (166, 366A), der ein Probeneinführungsende (169, 369A) umfasst, mit dem ersten Körperabschnitt (164, 362) an einer Verbindung verbunden ist und einen zweiten Fluiddurchgang in Fluidkommunikation mit dem ersten Fluiddurchgang definiert; und Merkmal (i) und (ii):
(i) die Verbindung umfasst ein Faltenbalggelenk (165, 365), das Schwenkbewegung zwischen dem ersten Körperabschnitt (164, 362) und dem zweiten Körperabschnitt (166, 366A) ermöglicht; und
(ii) der zweite Fluiddurchgang umfasst eine ungleichmäßige Breite zwischen dem Probeneinführungsende (169, 369A) und der Verbindung, mit einer Breitenabmessung an dem Probeneinführungsende (169, 369A), die größer als eine Breitenabmessung an der Verbindung ist;
Bewirken von Schwenkbewegung des Faltenbalggelenks (165, 365), um Ausrichtung zwischen einer zentralen Längsachse des zweiten Körperabschnittes (166, 366A) und einer zentralen Längsachse des ersten Körperabschnittes (164, 362) einzustellen;
Extrahieren des mindestens einen Materials aus dem Inneren des Zellkulturgehäuses in oder durch die Pipettenstruktur (160B, 360A'); und
Herausziehen des mindestens einen Teils der Pipettenstruktur (160B, 360A') aus dem Inneren des Zellkulturgehäuses (70).

8. Verfahren nach Anspruch 7, wobei das mindestens eine Material Zellkulturmedium (78) innerhalb des Inneren des Zellkulturgehäuses (70) umfasst und das Verfahren ferner Halten des Probeneinführungsendes (169, 369A) in einer Nichtkontaktbeziehung relativ zu der strukturierten Oberfläche (74) auf eine Weise umfasst, um bevorzugt Zellkulturmedium (78) aus dem Inneren des Zellkulturgehäuses (70) zu entfernen, während eine Wahrscheinlichkeit reduziert wird, dass Zellmehrzellaggregate aus der Reihe von Mikrovertiefungen (76) während des Extrahierens des mindestens einen Materials aus dem Inneren des Zellkulturgehäuses (70) entfernt werden.

9. Verfahren nach Anspruch 7, wobei das mindestens eine Material die Mehrzellaggregate umfasst und das Verfahren ferner Halten des Probeneinführungsendes nahe einer oder mehrerer ausgewählter Mikrovertiefungen (76) der Reihe von Mikrovertiefungen (76) auf eine Weise umfasst, um bevorzugt mindestens einige Mehrzellaggregate von der einen oder den mehreren Mikrovertiefungen (76) während des Extrahierens des mindestens einen Materials aus dem Inneren des Zellkulturgehäuses (70) zu entfernen.

10. Verfahren zum Herstellen der Pipettenstruktur nach Anspruch 1, wobei das Verfahren Folgendes umfasst:
Zuführen eines erhitzten Vorformlings zu einer Form;
Erzeugen eines Differenzdrucks zwischen einem Inneren und einem Äußeren des Vorformlings, um zu bewirken, dass sich der Vorformling ausdehnt und sich an einen Hohlraum der Form anpasst;
Öffnen der Form; und
Auswerfen der Pipettenstruktur aus der Form.

## Revendications

1. Structure de pipette (160, 160B, 180, 240, 260, 320, 340, 360, 360A') comprenant :
une première section de corps (164, 184, 242, 362) définissant un premier passage de fluide (174, 194, 256) ;
une seconde section de corps (166, 186, 246, 366A) comprenant une extrémité d'introduction d'échantillon, étant reliée à la première section de corps (164, 184, 242, 362) au niveau d'une jonction, et définissant un second passage de fluide (172, 192, 252) en communication fluidique avec le premier passage de fluide (174, 194, 256) ;
dans laquelle la structure de pipette comprend les caractéristiques (i) et (ii) :
(i) la jonction comprend un joint à soufflet (165, 185, 245, 365) permettant un mouvement de pivotement entre la première section de corps (164, 184, 242, 362) et la seconde section de corps (166, 186, 246, 366A) et une transition coudée non linéaire (168B, 328) agencée entre la première section de corps (164, 304) et la seconde section de corps (166, 327), dans laquelle la transition coudée non linéaire (166, 328) comprend un angle de transition dans une plage de 5 degrés à 60 degrés ; et
(ii) le second passage de fluide (172, 192, 252) comprend une largeur non uniforme entre l'extrémité d'introduction d'échantillon (169, 189, 249) et la jonction, avec une dimension de largeur à l'extrémité d'introduction d'échantillon (169, 189, 249) qui est supérieure à une dimension de largeur au niveau de la jonction.

2. Structure de pipette selon la revendication 1, réalisée dans une pipette de mesure unitaire (160, 180), dans laquelle :
la première section de corps (164, 184) comprend un corps tubulaire de la pipette de mesure (160, 180) ;
la seconde section de corps (166, 186) comprend une région de pointe (169, 189) de la pipette de mesure (160, 180) ; et
le corps tubulaire est agencé entre la région de pointe (169, 189) et une région d'embouchure (161, 181) de la pipette de mesure (160, 180).

3. Structure de pipette selon la revendication 1, mise en oeuvre dans un dispositif de pointe d'extension (240, 260) à utiliser avec une pipette de mesure (60), dans laquelle la première section de corps (242) définit une cavité (256, 246, 276) configurée pour recevoir une région de pointe de la pipette de mesure (60).

4. Structure de pipette selon la revendication 3, dans laquelle :
la cavité (276) est délimitée par une surface intérieure (277), et la surface intérieure (277) définit un évidement annulaire (278) configuré pour recevoir une bague d'étanchéité (279) pour favoriser l'étanchéité entre le dispositif de pointe d'extension (260) et la région de pointe de la pipette de mesure (60) ;
au moins une partie de la seconde section de corps (166A, 166B, 286) à proximité de l'extrémité d'introduction d'échantillon (169, 189, 249) comprend un axe longitudinal central (178, 289), et une pointe de l'extrémité d'introduction d'échantillon (169 , 189, 249) est coupée à un angle (β) non perpendiculaire à l'axe longitudinal central (178, 289) ;
la pointe de l'extrémité d'introduction d'échantillon (169, 189, 249) est découpée à un angle compris entre 5 degrés et 45 degrés par rapport à la perpendiculaire à l'axe longitudinal central (178) ;
une pointe de l'extrémité d'introduction d'échantillon (349) comprend une dimension de largeur primaire et une dimension de largeur transversale, dans laquelle la dimension de largeur primaire est au moins deux fois supérieure à la dimension de largeur transversale ;
la dimension de largeur primaire au niveau de l'extrémité d'introduction d'échantillon (369) est supérieure à la dimension de largeur au niveau de la jonction, et la dimension de largeur transversale au niveau de l'extrémité d'introduction d'échantillon (369) est inférieure à la dimension de largeur au niveau de la j onction ;
la première section de corps (164) et la seconde section de corps (166) comprennent au moins un matériau polymère ;
ou une combinaison de ceux-ci.

5. Structure de pipette selon l'une quelconque des revendications 1 à 4, comprenant en outre une interface soudée (165') entre la première section de corps (164) et la seconde section de corps (166) le long d'au moins une partie de la jonction.

6. Structure de pipette selon l'une quelconque des revendications 1 à 5, dans laquelle la première section de corps (164) et la seconde section de corps (166) sont configurées ou sont configurables pour permettre à un axe longitudinal central (178) de la seconde section de corps (166) d'être orienté selon un angle dans une plage de 100 degrés à 170 degrés par rapport à un axe longitudinal central (179) de la première section de corps (164).

7. Procédé de transfert d'au moins un matériau entre (i) un intérieur d'un logement de culture cellulaire (70) qui comprend une surface structurée (74) définissant un réseau de micropuits (76) adapté à la culture tridimensionnelle d'agrégats multicellulaires et (ii) un environnement extérieur au logement de culture cellulaire (70), le procédé comprenant :
l'insertion d'au moins une partie d'une structure de pipette (160B, 360A') à travers un orifice (85) du logement de culture cellulaire (70) dans l'intérieur du logement de culture cellulaire (70), dans lequel la structure de pipette (160B, 360A') comprend : une première section de corps (164, 362) définissant un premier passage de fluide ; une seconde section de corps (166, 366A) comprenant une extrémité d'introduction d'échantillon (169, 369A), étant reliée à la première section de corps (164, 362) au niveau d'une jonction, et définissant un second passage de fluide en communication fluidique avec le premier passage de fluide ; et les caractéristiques (i) et (ii) :
(i) la jonction comprend un joint à soufflet (165, 365) permettant un mouvement de pivotement entre la première section de corps (164, 362) et la seconde section de corps (166, 366A) ; et
(ii) le second passage de fluide comprend une largeur non uniforme entre l'extrémité d'introduction d'échantillon (169, 369A) et la jonction, avec une dimension de largeur au niveau de l'extrémité d'introduction d'échantillon (169, 369A) qui est supérieure à une dimension de largeur au niveau de la jonction d'extrémité ;
l'exécution d'un mouvement de pivotement du joint à soufflet (165, 365) pour ajuster l'orientation entre un axe longitudinal central de la seconde section de corps (166, 366A) et un axe longitudinal central de la première section de corps (164, 362) ;
l'extraction de l'au moins un matériau de l'intérieur du logement de culture cellulaire dans ou à travers la structure de pipette (160B, 360A') ; et
le retrait de l'au moins une partie de la structure de pipette (160B, 360A') de l'intérieur du logement de culture cellulaire (70).

8. Procédé selon la revendication 7, dans lequel l'au moins un matériau comprend un milieu de culture cellulaire (78) à l'intérieur du logement de culture cellulaire (70), et le procédé comprend en outre le maintien de l'extrémité d'introduction d'échantillon (169, 369A) dans une relation sans contact par rapport à la surface structurée (74) de manière à retirer préférentiellement le milieu de culture cellulaire (78) de l'intérieur du logement de culture cellulaire (70) tout en réduisant la probabilité de retrait des agrégats multicellulaires du réseau de micropuits (76) pendant ladite extraction de l'au moins un matériau de l'intérieur du logement de culture cellulaire (70).

9. Procédé selon la revendication 7, dans lequel l'au moins un matériau comprend les agrégats multicellulaires, et le procédé comprend en outre le maintien de l'extrémité d'introduction d'échantillon à proximité d'un ou plusieurs micropuits sélectionnés (76) du réseau de micropuits (76) de manière à retirer préférentiellement au moins certains agrégats multicellulaires de l'un ou plusieurs micropuits sélectionnés (76) pendant ladite extraction de l'au moins un matériau de l'intérieur du logement de culture cellulaire (70).

10. Procédé de fabrication de la structure de pipette selon la revendication 1, le procédé comprenant :
la fourniture d'une paraison chauffée à un moule ;
la création d'une pression différentielle entre un intérieur et un extérieur de la paraison pour amener la paraison à se dilater et à se conformer à une cavité du moule ;
l'ouverture du moule ; et
l'éjection de la structure de pipette du moule.
